# EUROPEAN PATENT SPECIFICATION

(11) **EP 1 040 094 B1**
(45) Date of publication and mention of the grant of the patent: **18.03.2009**
(21) Application number: 98966003.0
(22) Date of filing: 17.12.1998
(51) Int. Cl.: C07C 69/74, C07C 61/06, C07C 61/20, C07C 49/105, C07C 49/297, C07C 49/395, C07C 49/597, C07D 261/20, C07D 233/54, C07D 341/00

(54) **SUBSTITUTED CYCLOPENTANE AND CYCLOPENTENE COMPOUNDS USEFUL AS NEURAMINIDASE INHIBITORS**
SUBSTITUIERTE CYCLOPENTAN- UND CYCLOPENTEN-VERBINDUNGEN ALS NEURAMINIDASE-BLOCKER
COMPOSES DES CYCLOPENTANE ET CYCLOPENTENE CONVENANT COMME INHIBITEURS DE NEURAMINIDASE

(30) Priority: 17.12.1997 US 69956 P; 13.05.1998 US 85252 P
(43) Date of publication of application: 04.10.2000
(73) Proprietor: BIOCRYST PHARMACEUTICALS INC., Birmingham, Alabama 35244 (US)
(72) Inventor: BABU, Yarlagadda, S., Birmingham, AL 35226 (US); CHAND, Pooran, Birmingham, AL 35226 (US); MONTGOMERY, John, A., Birmingham, AL 35213 (US)
(74) Representative: Pellegri, Alberto
(86) International application number: PCT/US1998/026871
(87) International publication number: WO 1999/033781

(56) References cited:
- EP-A- 0 266 730
- WO-A-97/06157
- WO-A-97/47194
- US-A- 4 089 897
- US-A- 5 362 728
- US-A- 5 453 533
- US-A- 5 739 160
- CECH F. ET AL.: "Reaktionen polyvalenter jodverbindungen-V" TETRAHEDRON., vol. 31, no. 6, 1975, pages 605-612, XP002298509 NLELSEVIER SCIENCE PUBLISHERS, AMSTERDAM.
- PATENT ABSTRACTS OF JAPAN vol. 1995, no. 09, 31 October 1995 (1995-10-31) & JP 7 145120 A (KURARAY CO LTD), 6 June 1995 (1995-06-06)
- KAM B L ET AL: "CARBOCYCLIC SUGAR AMINES: SYNTHESIS AND STEREOCHEMISTRY OF RACEMIC ALPHA- AND BETA-CARBOCYCLIC RIBOFURANOSYLAMINE, CARBOCYCLIC LYXOFURANOSYLAMINE, AND RELATED COMPOUNDS" JOURNAL OF ORGANIC CHEMISTRY, AMERICAN CHEMICAL SOCIETY. EASTON, US, vol. 46, 1981, pages 3268-3272, XP002014506 ISSN: 0022-3263
- TAYLOR S J C ET AL: "CHEMOENZYMATIC SYNTHESIS OF (-)-CARBOVIR UTILIZING A WHOLE CELL CARALYSED RESOLUTION OF 2-AZABICYCLOU2.2.1HEPT-5-EN-3-ONE" JOURNAL OF THE CHEMICAL SOCIETY, CHEMICAL COMMUNICATIONS, CHEMICAL SOCIETY. LETCHWORTH, GB, 1990, pages 1120-1121, XP002063689 ISSN: 0022-4936
- TROST B.M. ET AL.: "An enantioselective synthesis of cis-4-tert-butoxycarbamoyl -1-methoxycarbonyl-2-cyclopentene" CHEMISTRY - A EUROPEAN JOURNAL., vol. 1, no. 8, 1995, pages 568-572, XP002298510 USVCH PUBLISHERS.
- DATABASE CHEMABS [Online] CHEMICAL ABSTRACTS SERVICE, COLUMBUS, OHIO, US; HUISGEN, ROLF ET AL: "1,3-Dipolar cycloadditions. 72. Reactions of fulminic acid with unsaturated compounds" XP002298512 retrieved from STN Database accession no. 80:27147 & CHEMISCHE BERICHTE , 106(10), 3291-311 CODEN: CHBEAM; ISSN: 0009-2940, 1973,
- DATABASE CHEMABS [Online] CHEMICAL ABSTRACTS SERVICE, COLUMBUS, OHIO, US; AKHREM, A. A. ET AL: "Synthesis and some properties of substituted 4,5-cyclopentano- .DELTA.2-isoxazolines" XP002298513 retrieved from STN Database accession no. 85:46478 & KHIMIYA GETEROTSIKLICHESKIKH SOEDINENII , (5), 625-8 CODEN: KGSSAQ; ISSN: 0132-6244, 1976,
- DATABASE CHEMABS [Online] CHEMICAL ABSTRACTS SERVICE, COLUMBUS, OHIO, US; DE AMICI, MARCO ET AL: "Nitrile oxides in medicinal chemistry. 6. Enzymic resolution of a set of bicyclic .DELTA.2-isoxazolines" XP002298514 retrieved from STN Database accession no. 125:10668 & TETRAHEDRON: ASYMMETRY , 7(3), 787-96 CODEN: TASYE3; ISSN: 0957-4166, 1996,
- DATABASE CHEMABS [Online] CHEMICAL ABSTRACTS SERVICE, COLUMBUS, OHIO, US; YANG, S. ET AL: "Resolution of .DELTA.2-isoxazoline-5-carboxylates by a protease from Aspergillus oryzae providing masked synthons for enantiopure .beta.-amino alcohols and related structures" XP002298515 retrieved from STN Database accession no. 121:133879 & MONATSHEFTE FUER CHEMIE , 125(4), 469-77 CODEN: MOCMB7; ISSN: 0026-9247, 1994,
- DATABASE CHEMABS [Online] CHEMICAL ABSTRACTS SERVICE, COLUMBUS, OHIO, US; KOROLEVA, E. V. ET AL: "Cleavage of 4,5-cyclopentisoxazolines by dimsylsodium" XP002298516 retrieved from STN Database accession no. 128:217309 & VESTSI AKADEMII NAVUK BELARUSI, SERYYA KHIMICHNYKH NAVUK , (3), 61-64 CODEN: VAKNEK; ISSN: 0002-3590, 1997,
- DATABASE CHEMABS [Online] CHEMICAL ABSTRACTS SERVICE, COLUMBUS, OHIO, US; LAKHVICH, F. A. ET AL: "Unusual hydrogenolysis of 2-isoxazolines" XP002298517 retrieved from STN Database accession no. 112:197661 & KHIMIYA GETEROTSIKLICHESKIKH SOEDINENII , (10), 1424-5 CODEN: KGSSAQ; ISSN: 0453-8234, 1989,
- DATABASE CHEMABS [Online] CHEMICAL ABSTRACTS SERVICE, COLUMBUS, OHIO, US; CURRAN, DENNIS P. ET AL: "Control of relative stereochemistry in the cycloadditive route to .beta.-hydroxy carbonyls. Regio- and stereoselective exo alkylation of .DELTA.2-isoxazolines" XP002298518 retrieved from STN Database accession no. 106:196311 & JOURNAL OF THE AMERICAN CHEMICAL SOCIETY , 109(10), 3036-40 CODEN: JACSAT; ISSN: 0002-7863, 1987,
- DATABASE CHEMABS [Online] CHEMICAL ABSTRACTS SERVICE, COLUMBUS, OHIO, US; CURRAN, DENNIS P. ET AL: "Thermolysis of bis[2-[(trimethylsilyl)oxy]propyl]furoxan (TOP-furoxan). The first practical method for intermolecular cycloaddition of an in situ generated nitrile oxide with 1,2-di- and trisubstituted olefins" XP002298519 retrieved from STN Database accession no. 103:160428 & JOURNAL OF THE AMERICAN CHEMICAL SOCIETY , 107(21), 6023-8 CODEN: JACSAT; ISSN: 0002-7863, 1985,
- DATABASE CHEMABS [Online] CHEMICAL ABSTRACTS SERVICE, COLUMBUS, OHIO, US; WADE, P. A. ET AL: "Benzenesulfonylcarbonitrile oxide. 4. Substitution reactions of 3-phenylsulfonylisoxazolines" XP002298520 retrieved from STN Database accession no. 98:215513 & JOURNAL OF ORGANIC CHEMISTRY , 48(11), 1796-800 CODEN: JOCEAH; ISSN: 0022-3263, 1983,
- DATABASE CHEMABS [Online] CHEMICAL ABSTRACTS SERVICE, COLUMBUS, OHIO, US; LAKHVICH, F. A. ET AL: "Approach to 7-ketoprostanoids via isoxazolines" XP002298521 retrieved from STN Database accession no. 111:39037 & KHIMIYA GETEROTSIKLICHESKIKH SOEDINENII , (7), 966-71 CODEN: KGSSAQ; ISSN: 0453-8234, 1988,
- DATABASE CHEMABS [Online] CHEMICAL ABSTRACTS SERVICE, COLUMBUS, OHIO, US; KWIATKOWSKI, STEFAN: "A new, efficient approach to .alpha.,.beta.-unsaturated ketones and .beta.-hydroxy ketones from 4,5-dihydroisoxazoles" XP002298522 retrieved from STN Database accession no. 108:220987 & JOURNAL OF THE CHEMICAL SOCIETY, CHEMICAL COMMUNICATIONS , (19), 1496-8 CODEN: JCCCAT; ISSN: 0022-4936, 1987,
- CANTRELL T.S. ET AL.: "The photolysis of 1,2,5-oxadiazoles, 1,2,5-thiadiazoles, and 2H-1,2,3-triazoles" CHEMICAL COMMUNICATIONS - CHEMCOM., no. 16, 1968, pages 977-978, XP002298511 GBROYAL SOCIETY OF CHEMISTRY.
- DATABASE CHEMABS [Online] CHEMICAL ABSTRACTS SERVICE, COLUMBUS, OHIO, US; ANTONEVICH, I. P. ET AL: "Synthesis of 2-acyl-1,1-ethylenedioxy-2-cyclopentenes" XP002298523 retrieved from STN Database accession no. 130:209441 & RUSSIAN JOURNAL OF ORGANIC CHEMISTRY (TRANSLATION OF ZHURNAL ORGANICHESKOI KHIMII) , 34(5), 676-679 CODEN: RJOCEQ; ISSN: 1070-4280, 1998,
- CHEMICAL ABSTRACTS, AN 110:39200, RAO et al., "Synthesis of Oxa- and Thia-Analogs of Bicyclo (n.3.0) Alkanes"; & INDIAN J. CHEM., Sect B, October 1987, Vol. 26B, No. 10, pages 939-946. XP002917986
- DATABASE CAPLUS, DN 129:161412, KLUENDER et al., "Derivatives of Substituted 4-Biarylbutyric Acid as Matrix Metalloprotease Inhibitors"; & US 5789434 A (BAYER CORPORATION) 04 August 1998. XP002917987
- DATABASE CAPLUS, DN 119:95005, OOMORI et al., "Preparation of Cyclic Enamino Esters as Intermediates for Antidepressants and Brian Disorder-Improving Agents"; & JP 05065255 A, 19 March 1993. XP002917988
- ALLAN R.D. et al, "Synthesis of Analogues of GABA, XV* Preparation and Resolution of Some Potent Cyclopentene and Cyclopentane Derivatives", AUST. J. CHEM., August 1986, Vol. 39, No. 6, pages 855-864. XP002917989
- DATABASE CAPLUS, DN 121:99068, SAKURAI et al., "Synthetic Study of HIV-1 Protease Inhibitors"; & PEPT. CHEM., January 1993, No. 31, pages 185-188. XP002917990

## Description

### Technical field

This invention relates to novel substituted cyclopentane compounds and derivatives thereof useful as neuraminidase inhibitors, and to pharmaceutical compositions containing said compounds useful for the prevention treatment or amelioration of viral, bacterial and other infections.

### Background of the invention

Despite the wealth of information available, influenza remains a potentially devastating disease of man, lower mammals, and birds. No effective vaccine exists and no cure is available once the infection has been initiated.

Influenza viruses consist of eight pieces of single stranded RNA, packaged in orderly fashion within the virion. Each piece codes for one of the major viral proteins. The replication complex is enclosed with a membrane composed of matrix protein associated with a lipid bilayer. Embedded in the lipid bilayer are two surface glycoprotein spikes, hemagglutinin (HA) and the enzyme neuraminidase (NA). All the viral genes have been cloned and the three dimensional structures of the surface glycoproteins have been determined.

Influenza viruses continually undergo antigenic variation in the two surface antigens, HA and NA, toward which neutralizing antibodies are directed. For this reason, vaccines and a subject's natural immune system have not been very effective. Attention is now being directed to finding other potential antiviral agents acting at others sites of the virion. This invention is directed to novel compounds which are useful in inhibiting the viral surface enzyme NA.

Furthermore, many other organisms carry also NA. Many of these NA-possessing organisms are also major pathogens of man and/or mammals, including *Vibraeo Cholerae, Clostridium perfringes, Streptococcus pneumonia, Arthrobacter sialophilas,* and other viruses, such as parainfluenza virus, mumps virus, newcastle disease virus, fowl plague virus, and Sendai virus. Compounds of this invention are also directed to inhibiting NA of these organisms.

In viruses, NA exists as a tetramer made of four roughly spherical subunits and a centrally-attached stalk containing a hydrophobic region by which it is embedded in the organism's membrane. Several roles have been suggested for NA. The enzyme catalyzes cleavage of the α-Ketosidic linkage between terminal sialic acid and the adjacent sugar residue. Removal of the sialic acid lowers the viscosity and permits access of the virus to the epithelial cells. NA also destroys the HA receptor on the host cell, thus allowing elution of progeny virus particles from infected cells.

Research indicates that the active site for influenza neuraminidase remains substantially unchanged for the major strains of influenza. For example, a comparison of sequences from influenza A subtypes and influenza B shows conserved residues with crucial structures and functional roles. Even though the sequence homology is only about 30 %, many of the catalytic residues are conserved. Furthermore, the three-dimensional structures of influenza A and B neuraminidases have been determined. Superposition of the various structures shows remarkable structural similarity of the active site. Since the active site amino acid residues are conserved in all known influenza A neuraminidases that have been sequenced so far, an inhibitor that is effective against different strains of influenza A and/or B neuraminidase can be designed based on the three dimensional structure of neuraminidase.

In general, the role of NA is thought to be for the mobility of the virus both to and from the site of infections. Compounds that inhibit neuraminidase's activity may protect a subject from infection and/or cure a subject once infection has set in. It is a further object of this invention to provide a method of using compounds of this invention for treating and/or curing a viral infection.

Analogues of neuraminic acid, such as 2-deoxy-2,3-didehydro-N-acetylneuraminic acid (DANA) and its derivatives are known to inhibit HA in vitro; however, these compounds are inactive in vivo. Palese and Schulman, IN CHEMOPROPHYLAXIX AND VIRUS INFECTIONS OF THE UPPER RESPIRATORY TRACT, Vol. 1 (J.S. Oxford, Ed.), CRC Press, 1977, at PS 189-205.

Von Itzstein et al. describes cyclohexane analogs of ∝-D-neuraminic acid of the formula 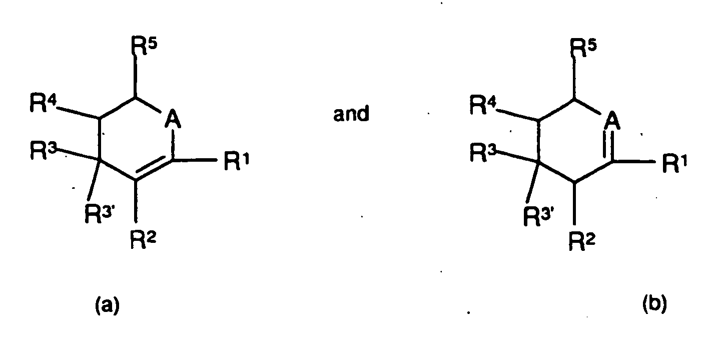 Wherein:
A is O, C or S in formula (a), and N or C in formula (b);
R¹ is CO₂H, PO₃H₂, NO₂, SO₂H, SO₃H, tetrazolyl-, CH₂CHO, CHO, or CH(CHO)₂;
R² is H, OR⁶, F, Cl, Br, CN, NHR⁶, SR⁶ or CH₂X, where X-is NHR⁶ halogen or OR⁶;
R³ and R^{3'} are H, CN, NHR⁶, SR⁶, = NOR⁶, OR⁶, guanidino, NR⁶;
R⁴ is NHR⁶, SR⁶, OR⁶, CO₂R⁶, NO₂, C(R⁶)₃, CH₂CO₂R⁶, CH₂NO₂ or CH₂NHR⁶;
R⁵ is CH₂YR⁶, CHYR⁶CH₂YR⁶ or CHYR⁶CHYR⁶CH₂YR⁶;
R⁶ is H, acyl, alkyl, allyl, or aryl;
Y is O, S, NH, or H;
and pharmaceutical salts thereof, useful as antiviral agents.

In addition, certain benzene derivatives are suggested in U.S. patent 5,453,533 as being inhibitors of influenza virus neuraminidase and various others are disclosed in U.S. patent No. 5,602,277. Yamamoto et al. describe various sialic acid isomers as having inhibitory activity against neuraminidase in Synthesis of Sialic Acid Isomers With Inhibitory Activity Against Neuraminidase, Tertrahedron Letters, Vol. 33, No. 39, pp. 5791-5794, 1992.

WO 96/26933 to Gilead sciences, Inc. describes certain 6-membered ring compounds as possible inhibitors of neuraminidase.

However, none of these references disclose the cyclopentane derivatives of the present invention.

### Summary of Invention

An aspect of the present invention is directed to compounds represented by the formula: 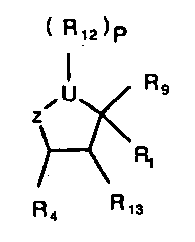 wherein
U is CH, and p is 1;
Z is -C(R₂)(R₃), -CH-N(R₂)(R₃), C(R₃)[(CH₂)nR₂], CH-C(R₃)(R₈)(CH₂)nR₂, C[(CH₂)nR₂]-[CH(R₃)(R₈)], C[(R₃)][CH[(CH₂)nR₂](R₈)];
R₁ is H, (CH₂)nOH, (CH₂)nNH₂, (CH₂)nNR₁₀ R₁₁, (CH₂)nOR₁₁, (CH₂)nSR₁₁, or (CH₂)n halogen
R₉ is (CH₂)nCO₂H_{,} (CH₂)nSO₃H, or (CH₂)nPO₃H₂, esters thereof, or salts thereof;
R₂ is H, NHC(O)R₅, NHC(S)R₅, NHSO₂R₅, C(O)NHR₅, SO₂NHR₅, CH₂S(O)R₅, or CH₂SO₂R₅
R₃ and R₈ individually is H, (CH₂)nC(O)R₁₀(CH₂)nCO₂R₁₀, (CH₂)nOR₁₀, CH(OR₁₀)CH(R₁₀)m, C(O)N(R₁₀)m, C(O)N(OR₁₀)R₁₀, (CH₂)nN(R₁₀)m, CH(R₁₀)m when m is 2, (CH₂)n(R₁₀)m, CH₂CH(OR₁₀)CH₂OR₁₀, CH(OR₁₀)CH(OR₁₀)CH₂OR₁₀, CH₂OR₁₀, CH(OR₁₀)CH₂NHR₁₀, CH₂CH(OR₁₀)CH₂NHR₁₀, CH(OR₁₀)CH(OR₁₀)CH₂NHR₁₀, C(=NR₁₀)N(R₁₀)m, NHR₁₀, or NHC(=NR₁₀)N(R₁₀)m
R₄ is H, (CH₂)nOH, (CH₂)nOR₁₁, (CH₂)nOC(O)R₁₁, (CH₂)nNHC(NR₁₁)NHR₁₁, (CH₂)nNR₁₀R₁₁, (CH₂)nNH₂, (CH₂)nC(=NH)(NH₂), (CH₂)nNHC(=NR₁₁)NH₂, (CH₂)nNHC(=NR₇)NH₂, (CH₂)nCN, (CH₂)nN₃, C(=NH)NH₂, C(=NR₇)NH₂, or C(=NR₁₁)NH₂;
R₅ is H, lower alkyl having 1 to 8 carbon atoms, cyclo alkyl, halogen substituted alkyl, aryl, substituted aryl, or CF₃;
R₇ is H, (CH₂)nOH, (CH₂)nCN, (CH₂)nNH₂, or (CH₂)nNO₂;
R₁₀ is H, lower alkyl having 1 to 8 carbon atoms, lower alkenyl having 2 to 8 carbon atoms, branched alkyl, cyclic alkyl, (CH₂)n aromatic, (CH₂)n substituted aromatic, or when m is 2 both R₁₀ groups can also be interconnected to form an N substituted heterocyclic ring, or other 5 or 6 membered heterocyclic ring;
R₁₁ is lower alkyl having 1 to 8 carbon atoms, branched alkyl, (CH₂)m aromatic, SO₂R₁₀, C(O)R₁₀, or C(O)OR₁₀;
R₁₂ is (CH₂)nOH, and R₁₃ is H;
m is 1 or 2;
n is 0-4;
and pharmaceutically acceptable salts thereof.

The present invention is also concerned with compositions for inhibiting influenza virus neuraminidase comprising a pharmaceutically acceptable carrier and an amount effective for inhibiting influenza virus neuraminidase of a compound as defined above.

### Best and various modes for carrying out Invention

An aspect of the present invention is directed to compounds represented by the formula: 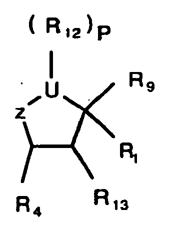 wherein
U is CH, and p is 1;
Z is -C(R₂)(R₃), -CH-N(R₂)(R₃), C(R₃)[(CH₂)nR₂], CH-C(R₃)(R₈)(CH₂)nR₂, C[(CH₂)nR₂]-[CH(R₃)(R₈)], C[(R₃)][CH[(CH₂)nR₂](R₈)];
R₁ is H, (CH₂)nOH, (CH₂)nNH₂, (CH₂)nNR₁₀ R₁₁, (CH₂)nOR₁₁, (CH₂)nSR₁₁, or (CH₂)n halogen
R₉ is (CH₂)nCO₂H, (CH₂)nSO₃H, or (CH₂)nPO₃H₂, esters thereof, or salts thereof;
R₂ is H, NHC(O)R₅, NHC(S)R₅, NHSO₂R₃, C(O)NHR₅, SO₂NHR₅, CH₂S(O)R₅, or CH₂SO₂R₅
R₃ and R₈ individually is H, (CH₂)nC(O)R₁₀, (CH₂)nCO₂R₁₀, (CH₂)nOR₁₀, CH(OR₁₀)CH(R₁₀)m, C(O)N(R₁₀)m, C(O)N(OR₁₀)R₁₀, (CH₂)nN(R₁₀)m, CH(R₁₀)m when m is 2, (CH₂)n(R₁₀)m, CH₂CH(OR₁₀)CH₂OR₁₀, CH(OR₁₀)CH(OR₁₀)CH₂OR₁₀, CH₂OR₁₀, CH(OR₁₀)CH₂NHR₁₀, CH₂CH(OR₁₀)CH₂NHR₁₀, CH(OR₁₀)CH(OR₁₀)CH₂NHR₁₀, C(=NR₁₀)N(R₁₀)m, NHR₁₀, or NHC(=NR₁₀)N(R₁₀)m;
R₄ is H, (CH₂)nOH, (CH₂)nOR₁₁, (CH₂)nOC(O)R₁₁, (CH₂)nNHC(NR₁₁)NHR₁₁, (CH₂)nNR₁₀R₁₁, (CH₂)nNH₂, (CH₂)nC(=NH)(NH₂), (CH₂)nNHC(=NR₁₁)NH₂, (CH₂)nNHC(=NR₇)NH₂, (CH₂)nCN, (CH₂)nN₃, C(=NH)NH₂, C(=NR₇)NH₂, or C(=NR₁₁)NH₂;
R₅ is H, lower alkyl, cyclo alkyl, halogen substituted alkyl, aryl, substituted aryl, or CF₃;
R₇ is H, (CH₂)nOH, (CH₂)nCN, (CH₂)nNH₂, or (CH₂)nNO₂;
R₁₀ is H, lower alkyl, lower alkenyl, branched alkyl, cyclic alkyl, (CH₂)n aromatic, (CH₂)n substituted aromatic, or when m is 2 both R₁₀ groups can also be interconnected to form an N substituted heterocyclic ring, or other 5 or 6 membered heterocyclic ring;
R₁₁ is lower alkyl, branched alkyl, (CH₂)m aromatic, SO₂R₁₀, C(O)R₁₀, or C(O)OR₁₀;
R₁₂ is (CH₂)nOH, and R₁₃ is H;
m is 1 or 2;
n is 0 - 4;
and pharmaceutically acceptable salts thereof.

The lower alkyl groups contain 1 to 8 carbon, and preferably 1 to 3 carbon atoms, and can be straight; branched-chain or cyclic saturated aliphatic hydrocarbon groups.

Examples of suitable alkyl groups include methyl, ethyl, and propyl. Examples of branched alkyl groups include isopropyl and t-butyl. Examples of suitable cyclic aliphatic groups typically contain 3-8 carbon atoms and include cyclopentyl and cyclohexyl. The aromatic or aryl groups are preferably phenyl or alkyl substituted aromatic groups (aralkyl) such as phenyl C₁₋₃ alkyl such as benzyl, or halo substituted aryl groups.

Examples of substituted cycloalkyl groups include cyclic aliphatic groups typically containing 3-8 carbon atoms in the ring substituted with alkyl groups typically having 1-6 carbon atoms and/or hydroxy group. Usually 1 or 2 substituted groups are present.

The esters are typically lower alkyl esters having 1 to 12 carbon atoms and preferably 1 to 3 carbon atoms and aryl esters containing 6 to 14 carbon atoms. The alkyl esters can be straight-chain, branched-chain or cyclic saturated aliphatic hydrocarbons.

Examples of some alkyl esters are methyl, ethyl, propyl, isopropyl, t-butyl, cyclopentyl and cyclohexyl esters. The aryl esters are preferably phenyl or alkyl substituted aromatic esters (alkaryl) including C₁₋₃ alkyl substituted phenyl such as benzyl.

The lower alkenyl group can be straight, branched chain or cyclic unsaturated hydrocarbon group and contains 2-8 carbon atoms and preferably 2-3 carbon atoms. Examples of alkenyl groups are vinyl, 1-propenyl, allyl, isopropenyl, 2-methyl-2-propenyl and cyclopentenyl.

The N-heterocyclic rings contain 3-7 atoms in the ring. The heterocyclic rings can be substituted such as with a lower alkyl group. Examples of suitable heterocyclic groups are pyrrolidino, azetidino, piperidino, 3,4-didehydropiperidino, 2-methylpiperidino and 2-ethylpiperidino.

As used herein, the term "hydrocarbon group" includes saturated and unsaturated straight or branched hydrocarbon groups, including aryl groups, and combinations of such groups.

Pharmaceutically acceptable salts of the compounds of formula (I) include those derived from pharmaceutically acceptable, inorganic and organic acids and bases. Examples of suitable acids include hydrochloric, hydrobromic, sulfuric, nitric, perchloric, fumaric, maleic, phosphoric, glycollic, lactic, salicylic, succinic, toluene-p-sulphonic, tartaric, acetic, citric, methanesulphonic, formic, benzoic, malonic, naphthalene-2-sulphonic, trifluoroacetic and benzenesulphonic acids.

Salts derived from appropriate bases include alkali such as sodium and ammonia.

It is of course understood that the compounds of the present invention relate to all optical isomers and stereo-isomers at the various possible atoms of the molecule.

Examples of some particular formulae within the scope of this invention are represented by the following:

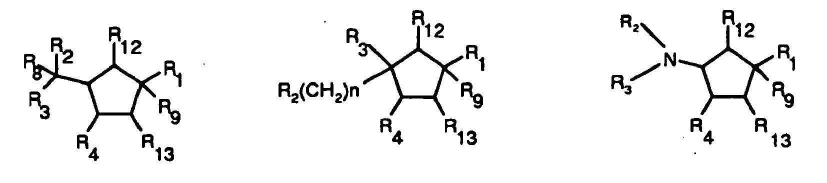

The naming system which has been used herein for the compounds of the following type is as follows:

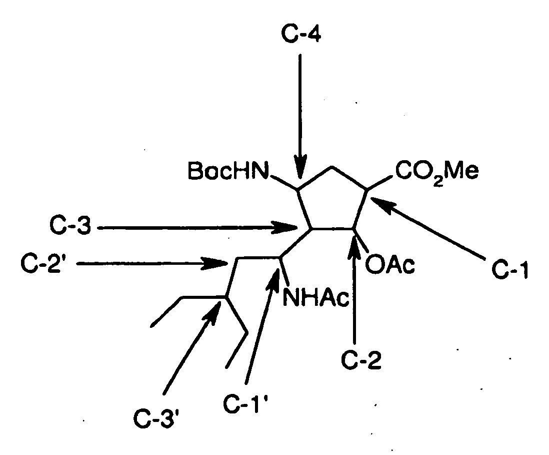

In some cases a and P have been used to show that these groups are trans to each other. These are the cases when we have more than two substituents on cyclopentane ring and only two are fixed.

In fused cyclopent[d]isoxazole system, the numbering is as follows:

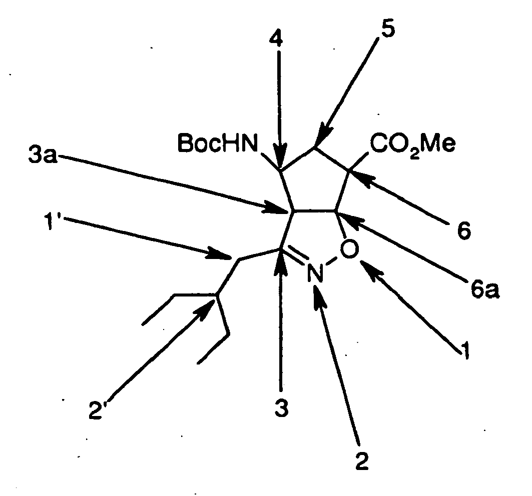

Examples of some specific compounds and intermediates according to the present application are identified in Examples 1, 2, 3, 4, 5, 6, 7, 8, 9, 10, 11, 12, 13,14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72.

The following schemes illustrate methods for preparing compounds of the present invention.

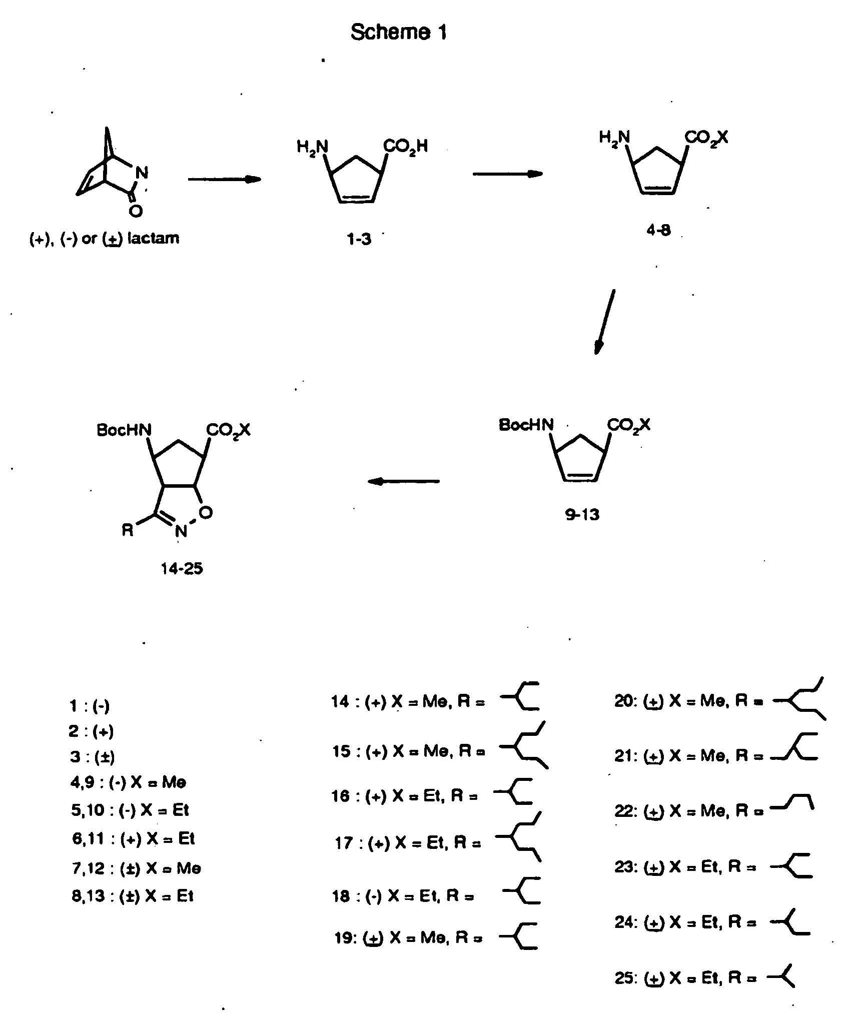

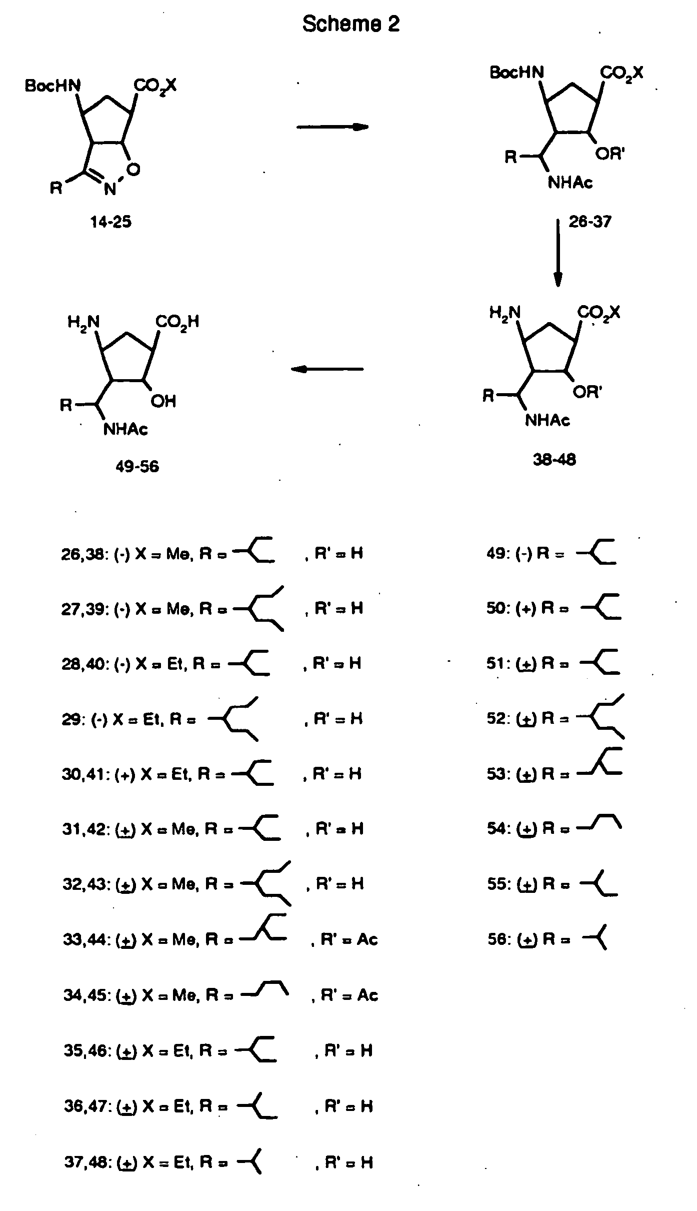

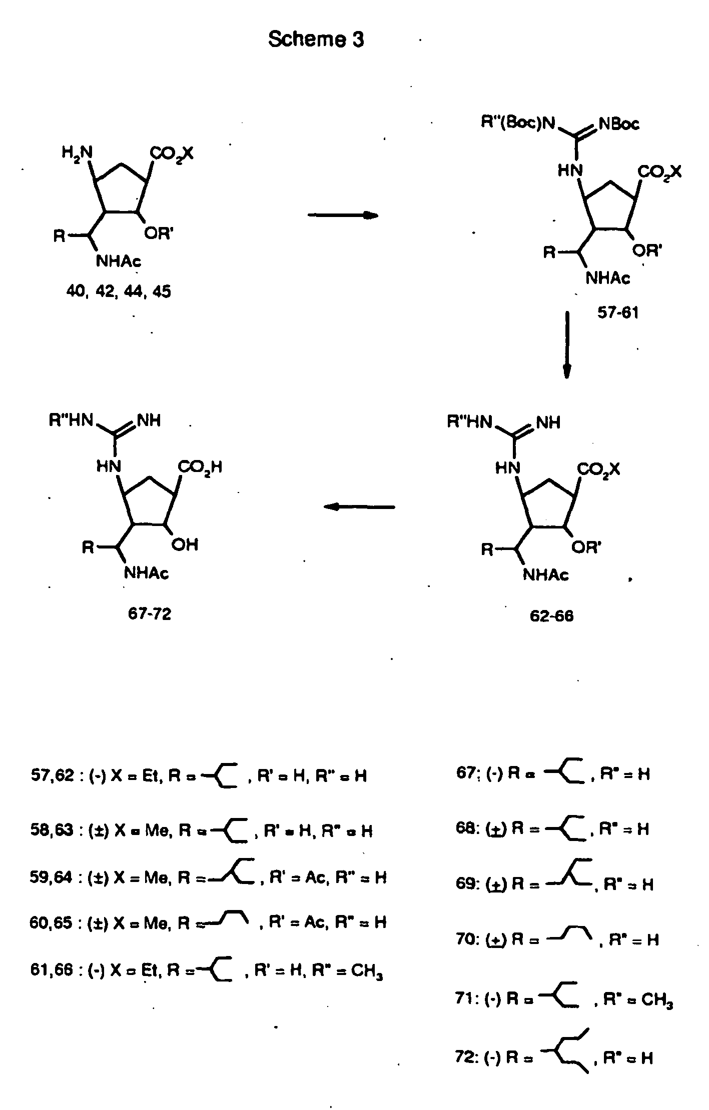

In addition, an important intermediate to the present invention, compounds 14-25, can be prepared by reacting a cyclopentene of the formula 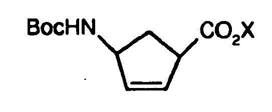 with corresponding nitrile oxide (produced from phenyl isocyanate, and a nitroalkane in the presence of triethylamine or from chloro-oxime and triethylamine) to produce an cyclopent[d]isoxazole ring system. The product can then be hydrogenated in the presence of a PtO₂ in an alcohol along with HCl to open the ring and form a corresponding amino compound.

Also, process for preparing compounds of the present invention can be found in U.S. Patent Application Serial No. 60/085,252 filed May 13 1998, entitled " Preparation of substituted Cyclopentane Compounds and Certain Intermediates" to Chand et al.

The following non-limiting examples are presented to further illustrate the present invention.

### Example 1

### (1S,4R)-(-)-4-Aminocyclopent-2-en-1-carboxylic acid Hydrochloride (1, Scheme 1)

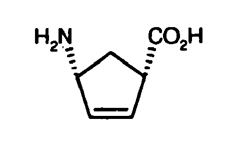

A mixture of (-)-(1*R*,4*S*)-2-azabicyclo[2.2.1]hept-5-en-3-one ((-)-lactam, 15 g, 137 mmol) and IN HCl (375 mL) was heated at reflux for 1h. The mixture was concentrated and the residue dried *in vacuo* to yield 1 in 95% yield. It was used as such for the next step.

### Example 2

### (1R,4S)-(+)-4-Aminocyclopent-2-en-1-carboxylic acid Hydrochloride (2, Scheme 1)

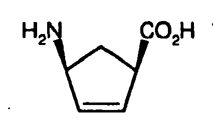

It was prepared from (+)-(1*S*,4*R*)-2-azabicyclo[2.2.1]hept-5-en-3-one ((+)-lactam, 4.9 g) according to the method used for compound 1.

### Example 3

### (±)-cis-4-Aminocyclopent-2-en-1-carboxylic acid Hydrochloride (3, Scheme 1)

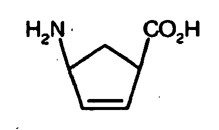

It was prepared from (±)-2-azabicyclo[2.2.1]hept-5-en-3-one ((±)-lactam, 3.2 g) according to the method used for compound 1.

### Example 4

### (1S,4R)-(-)-Methyl-4-aminocyclopent-2-en-1-carboxylate Hydrochloride (4, Scheme 1)

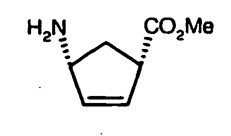

A mixture of (-)-(1*R*,4*S*)-2-azabicyclo[2.2.1]hept-5-en-3-one ((-)-lactam, 600 g, 5.51 mol) and IN methanolic HCl (12 L) was heated at reflux for 10 h. The solvent was evaporated under reduced pressure and to the residue was added ether (800 mL), and cooled. The solid obtained was collected by filtration, washed with ether and dried to give 956 g (98%) of compound 4, as white crystalline solid, mp 106-108 °C.
¹H NMR (DMSO-d₆): δ 1.98 (m, 1 H), 2.52 (m, 1 H), 3.6 (s, 3 H), 3.68 (m, 1 H), 4.15 (m 1 H), 5.88 (d, 1 H), 6.08 (d, 1 H), 8.40 (m, 2 H); MS (ES+): 142.11 (100%, M+1); IR (KBr): 3004, 1722, 1239, 1217 cm-¹.

| | | |
|---|---|---|
| Analysis: | Calcd for C₇H₉NO₂: | C, 47.33; H, 6.81; N, 7.89 |
| | Found: | C, 47.12; H, 7.12; N, 7.85 |

### Example 5

### (1S,4R)(-)-Ethyl-4-aminocyclopent-2-en-1-carboxylate Hydrochloride (5, Scheme 1)

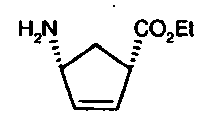

Method A: It was prepared from (-)-(1*R*,4*S*)-2-azabicyclo[2.2.1]hept-5-en-3-one ((-)-lactam, 4.9 g) and ethanolic HCl according to the method used for compound 4.
Method B: It was also prepared from compound 1 and ethanoil HCl according to the method used for compound 4.

### Example 6

### (1R,4S)-(+)-Ethyl-4-aminocyclopent-2-en-1-carboxylate Hydrochloride (6, Scheme 1)

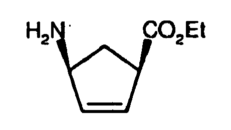

It was prepared from compound 2 (7.3 g) according to the method-B used for compound 5.

### Example 7

### (±)-cis-Methyl-4-aminocyclopent-2-en-1-carboxylate Hydrochloride (7, Scheme 1)

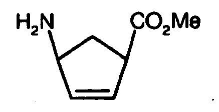

It was prepared from (±)-2-azabicyclo[2.2.1]hept-5-en-3-one ((±)-lactam, 11.2 g) and methanolic HCl according to the method used for compound 4.

### Example 8

### (±)-cis-Ethyl-4-aminocyclopent-2-en-1-carboxylate Hydrochloride (8, Scheme 1)

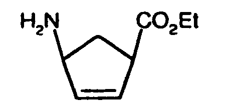

It was prepared from 3 (4.9 g) and ethanolic HCl according to the method-B used for compound 5.

### Example 9

### (1S,4R)-(-)-Methyl-4-tert-butoxycarbonylaminocyclopent-2-en-1-carboxylate (9, Scheme 1)

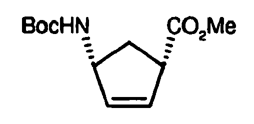

To a mixture of compound 4 (950 g, 5.35 mol) and di-tert-butyldicarbonate (1226 g, 5.62 mol) in dichloromethane (12 L) at 0 °C was added triethylamine over a period of 2.5 h and the reaction mixture further stirred for 1 h. The reaction mixture was washed with water (2x 8 L) and brine (2x 4 L) and the organic layer separated and dried over MgSO₄. After filtration, the filtrate was concentrated and the residual thick syrup was crystallized from hexane to give 1196 g (92%) of compound 9 in 3 crops.
¹H NMR (CDCl₃): δ 1.44 (s, 9 H), 1.85 (m, 1H), 2.51 (m 1 H), 3.47 (m, 1 H), 3.71 (s, 3 H), 4.78 (m, 1 H), 4.88 (br s, 1 H), 5.86 (m, 2 H); MS (ES+): 242.25 (80%, M+1).

| | | |
|---|---|---|
| Analysis: | Calcd for C₁₂H₁₉NO₄: | C, 59.73; H, 7.94; N, 5.80 |
| | Found: | C, 59.57; H, 7.86; N, 5.79 |

### Example 10

### (1S,4R)-(-)-Ethyl-4-tert-butoxycarbonylaminocyclopent-2-en-1-carboxylate (10, Scheme 1)

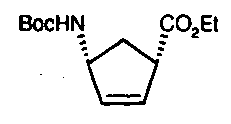

It was prepared from 5 (17.5 g) according to the method used for compound 9.

### Example 11

### (1R,4S)-(+)-Ethyl-4-tert-butoxycarbonylaminocyclopent-2-en-1-carboxylate (11, Scheme 1)

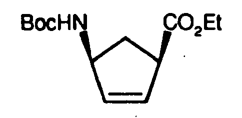

It was prepared from 6 (8.4 g) according to the method used for compound 9.

### Examples 12

### (±)-cis-Methyl-tert-butoxycarbonylaminocyclopent-2-en-1-carboxylate (12, Scheme 1)

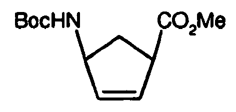

It was prepared from 7 (17.8 g) according to the method used for compound 9.

### Example 13

### (±)-cis-Ethyl-4-tert-butoxycarbonylaminocyclopent-2-en-1-carboxylate (13, Scheme 1)

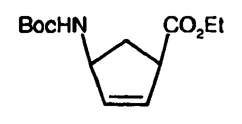

It was prepared from 8 (20.8 g) according to the method used for compound 9.

### Example 14

### (3aR,4R,6S,6aS)-(+)-Methyl-4-tert-butoxycarbonylamino-3-(1'-ethylpropyl)-4,5,6,6a-tetrahydro-3aH-cyclopent[d]isoxazole-6-carboxylate (14, Scheme 1)

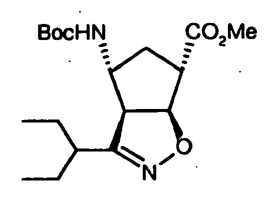

Method-A: A mixture of 9 (949 g, 3.93 mol) and phenyl isocyanate (1.5 L, 13.8 mol) in benzene (10 L) was heated under reflux. To this mixture was added, a mixture of 2-ethyl-1-nitrobutane (918 g, 5.92 mol, 85 % pure by ¹H NMR) and triethylamine (55 mL, 0.40 mol) in benzene (2 L) over a period of 5 h. The reaction mixture was further stirred under reflux for 24 h. On cooling, the solids were removed by filtration, the filtrate was concentrated and to the residue was added ethyl ether (2 L). The mixture was allowed to stand overnight, the solids were again removed by filtration and the filtrate was concentrated *in vacuo* to give 1.991 Kg of a dark brown syrup.

The product obtained above was dissolved in THF (5.0 L) and ethanol (7.5 L). To this mixture was added, NaOH (454 g in 5 L cold water) and the mixture stirred at room temperature for 3 h. The mixture was then concentrated *in vacuo.* The residue was dissolved in water (10 L), extracted with ethyl ether (2 x 2 L) and the organic layers were discarded. The aqueous layer was acidified with acetic acid and extracted with ethyl acetate (2 x 7 L). The combined organic extracts were dried (MgSO₄) and the organic layer concentrated to give 1.75 Kg of the crude acid.

To the above product in methanol (19.5 L) was added conc. HCl (162 mL) and the mixture stirred at room temperature for 16 h. The mixture was neutralized with ammonium hydroxide and the solvent evaporated *in vacuo* to give 1.422 Kg of the crude ester. The crude product was dissolved in ethyl acetate (5 L), washed with water (5 L) and brine (5 L), and dried (MgSO₄). After filtration, the filtrate was concentrated and the residue was purified by passing through a column of silica gel using hexane/ethyl acetate (95/5 to 85/15) mixture as the eluent. The appropriate fractions were pooled together and concentrated to give 984 g (70.5 %) of 14, mp 66 °C.

| | | |
|---|---|---|
| Analysis: | Calcd for C₁₈ H₃₀N₂O₅: | C, 61.00; H, 8.53; N, 7.90 |
| | Found: | C, 61.13; H, 8.45; N, 7.84 |

¹H NMR (CDCl₃): δ ppm 0.87- 0.95 (m, 6H), 1.44 (s, 9H), 1.58-1.78 (m, 4H), 2.0-2.15 (m, 2H), 2.51 (b s, 1H), 3.21 (d, J=8.1 Hz, 1H), 3.58 (d, J=8.8 Hz, 1H), 3.76 (s, 3H), 4.23 (b s, 1H), 5.21 (d, 1H, J=8.8 Hz), 5.59 (b s, 1H); MS (ES+): 355.64 (M+1).
Method-B: The reaction of cyclopentene compound 9 with 1-nitro-2-ethylbutane and phenyl isocyanate was done the same way as described in method-A. After the reaction was complete (24 h reflux), the solids were removed by filtration, and the filtrate was concentrate. The residue was purified two times by passing through a silica gel column using ethyl acetate: hexane mixture as eluent.
Method-C: A mixture of 9 (1.08 Kg, 4.46 mol) and 2-ethylbutyrohydroximinoyl chloride (prepared from 2-ethylbutyraldoxime and N-chlorosuccinimide, 614 g, 4.1 mol) in THF (8 L) was heated at reflux. To this mixture was added a mixture of triethylamine (340 mL, 2.4 mol) in THF over a period of 1.5 h. Additional amount of chloro-oxime (550 g, 3.7 mol) was added followed by a mixture of triethylamine (340 mL, 2.4 mol) in THF (340 mL) over 1 h period. This exact addition was repeated twice. Additional triethylamine (100 mL, 0.7 mol) was added in 30 min. The reaction mixture was further heated at reflux for 16 h. The reaction mixture was cooled and the insoluble solid was removed by filtration. The filtrate was concentrated and the residue was purified either by chromatography or through acid-base treatment as described in methods A and B.

### Example 15

### (3aR,4R,6S,6aS)-(+)-Methyl-4-tert-butoxycarbonylamino-3-(1'-propylbutyl)-4,5,6,6a-tetrahydro-3aH-cyclopent[d]isoxazole-6-carboxylate (15, Scheme 1)

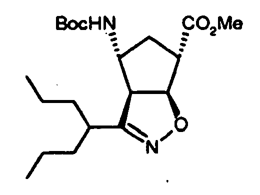

It was prepared from 9 (171 g) using the same procedure as for compound 14 (Method-A).

### Example 16

### (3aR,4R,6S,6aS)-(+)-Ethyl-4-tert-butoxycarbonylamino-3-(1'-ethylpropyl)-4,5,6,6a-tetrahydro-3aH-cyclopent[d]isoxazole-6-carboxylate (16, Scheme 1)

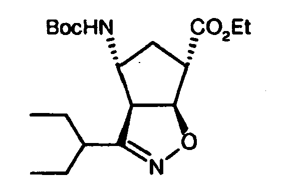

It was prepared from 10 (5.64 g) using the same procedure as for compound 14 (Method-B).

### Example 17

### (3aR,4R,6S,6aS)-(+)-Ethyl-4-tert-butoxycarbonylamino-3-(1'-propylbutyl)-4,5,6,6a-tetrahydro-3aH-cyclopent[d]isoxazole-6-carboxylate (17, Scheme 1)

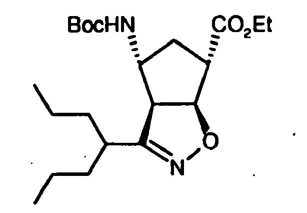

It was prepared from 10 (16.1 g) using the same procedure as for compound 14 (Method-B).

### Example 18

### (3aS,4S,6R,6aR)-(-)-Ethyl-4-tert-butoxycarbonylamino-3-(1'-ethylpropyl)-4,5,6,6a-tetrahydro-3aH-cyclopent[d]isoxazole-6-carboxylate (18, Scheme 1)

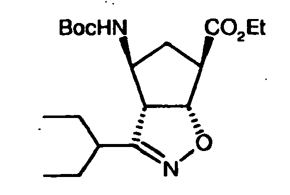

It was prepared from 11 (5.1 g) using the same procedure as for compound 14 (Method-B).

### Example 19

### (±)-Methyl-4-tert-butoxycarbonylamino-3-(1'-ethylpropyl)-4,5,6,6a-tetrahydro-3aH-cyclopent[d]isoxazole-6-carboxylate (NHBoc and ester groups cis to each other but trans to isooxazoline ring, 19, Scheme 1)

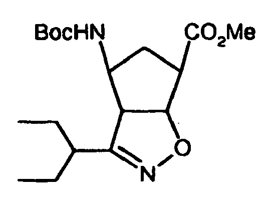

It was prepared from 12 (4.8 g) using the same procedure as for compound 14 (Method-B).

### Example 20

### (±)-Methyl-4-tert-butoxycarbonylamino-3-(1'-propylbutyl)-4,5,6,6a-tetrahydro-3aH-cyclopent[d]isoxazole-6-carboxylate (NHBoc and ester groups cis to each other but trans to isooxazoline ring, 20, Scheme 1)

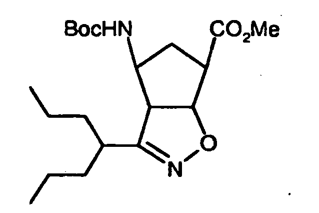

It was prepared from 12 (4.0 g) using the same procedure as for compound 14 (Method-B).

### Examples 21

### (±)-Methyl-4-tert-butoxycarbonylamino-3-(2'-ethylbutyl)-4,5,6,6a-tetrahydro-3aH-cyclopent[d]isoxazole-6-carboxylate (NHBoc and ester groups cis to each other but trans to isooxazoline ring, 21, Scheme 1)

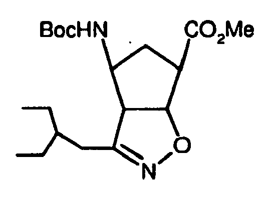

It was prepared from 12 (1.18 g) using the same procedure as for compound 14 (Method-B).

### Example 22

### (±)-Methyl-4-tert-butoxycarbonylamino-3-(n-butyl)-4,5,6,6a-tetrahydro-3aH-cyclopent[d]isoxazole-6-carboxylate (NHBoc and ester groups cis to each other but trans to isooxazoline ring, 22, Scheme 1)

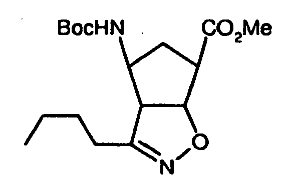

It was prepared from 12 (1.2 g) using the same procedure as for compound 14 (Method-B).

### Example 23

### (±)-Ethyl-4-tert-butoxycarbonylamino-3-(1'-ethylpropyl)-4,5,6,6a-tetrahydro-3aH-cyclopent[d]isoxazole-6-carboxylate (NHBoc and ester groups cis to each other but trans to isooxazoline ring, 23, Scheme 1)

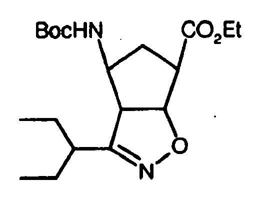

It was prepared from 13 (4.8 g) using the same procedure as for compound 14 (Method-B).

### Example 24

### (±)-Ethyl-4-tert-butoxycarbonylamino-3-(1'-methylpropyl)-4,5,6,6a-tetrahydro-3aH-cyclopent[d]isoxazole-6-carboxylate (mixture at C-1', NHBoc and ester groups cis to each other but trans to isooxazoline ring, 24, Scheme 1)

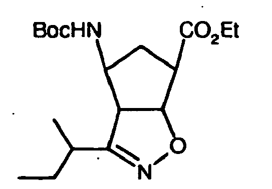

It was prepared from 13 (3.5 g) using the same procedure as for compound 14 (Method-B).

### Example 25

### (±)-Ethyl-4-tert-butoxycarbonylamino-3-(1'-methylethyl)-4,5,6,6a-tetrahydro-3aH-cyclopent[d]isoxazole-6-carboxylate (NHBoc and ester groups cis to each-other but trans to isooxazoline ring, 25, Scheme 1)

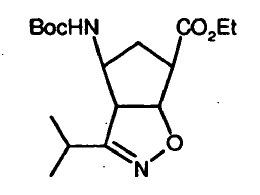

It was prepared from 13 (2.9 g) using the same procedure as for compound 14 (Method-B).

### Example 26

### (1S,2S,3R,4R,1'S)-(-)-Methyl 3-(1'-acetylamino-2'-ethyl)butyl-4-tert-butoxycarbonylamino-2-hydroxycyclopentan-1-carboxylate (26, Scheme 2)

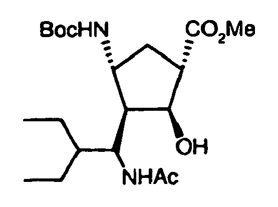

To a mixture of 14 (80 g, 0.226 mol) in methanol (1.6 L) were added conc. HCl (18.8 mL, 0.226 mol) and Adam's catalyst (PtO₂, 8.0 g) and the mixture was stirred very vigorously at 100 psi hydrogen pressure for 4 h. The catalyst was removed by filtration and the filtrate concentrated to give 82.7 g (93 %) of (1*S*,2*S*,3*R*,4*R*,1'*S*)-(-)-Methyl 3-(1'-amino-2'-ethyl)butyl-4-*tert*-butoxycarbonyl- amino-2-hydroxycyclopentan-1-carboxylate, which was used as such for acetylation.

To the above obtained amine hydrochloride (66.2 g, 0.168 mol) in dichloromethane (600 mL), were added triethylamine (23.4 mL, 0.168 mol) and acetic anhydride (17.5 mL, 0.184 mol) at room temperature and stirred for 2 h. The reaction mixture was washed with water (600 mL). Water layer was back extracted with dichloromethane (200 m). The combined organic layers were washed with water (300 mL) and brine (300 mL), and dried over magnesium sulfate. After filtration, the filtrate was concentrated and the residue purified by passing through a column of silica gel using hexane/ ethyl acetate (1:1) as an eluent. The appropriate fractions were pooled together and concentrated to give 45 g (67%) of compound 26.

| | | |
|---|---|---|
| Analysis: | Calcd for C₂₀ H₃₆N₂O₆· | C, 59.98; H, 9.06; N, 6.99 |
| | Found: | C, 59.89; H, 8.91; N, 6.94 |

¹H NMR (CDCl₃): δ ppm 0.77- 0.87 (m, 6H), 1.19- 1.44 (m, 15H), 1.66-1.72 (m, 1H), 1.96-2.00 (m, 1H), 2.08 (s, 3H), 2.45-2.53 (m, 1H), 2.80-2.84 (m, 1H), 3.70 (s, 3H), 3.99-4.04 (m, 1H), 4.11-4.15 (m, 1H), 4.23 (d, J=5.2 Hz, 1H), 4.78 (d, J=9.3 Hz, 1H), 7.55 (d, J=10.0 Hz, 1H); MS (ES+): 401.75 (M+1).

### Example 27

### (1S,2S,3R,4R,1'S)-(-)-Methyl 3-(1'-acetylamino-2'-propyl)pentyl-4-tert-butoxycarbonyl- amino-2-hydroxycyclopentan-1-carboxylate (27, Scheme 2)

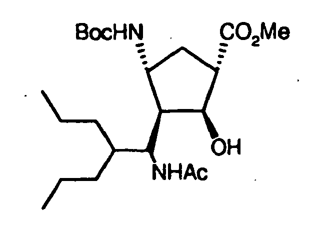

It was prepared from 15 (77 g) using the same procedure as for compound 26.

### Example 28

### (1S,2S,3R,4R,1'S)-(-)-Ethyl 3-(1'-acetylamino-2'-ethyl)butyl-4-tert-butoxycarbonylamino-2-hydroxycyclopentan-1-carboxylate (28, Scheme 2)

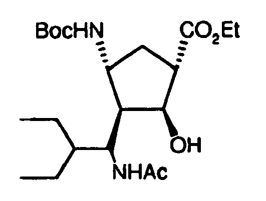

It was prepared from 16 (5 g) using the same procedure as for compound 26.

### Example 29

### (1S,2S,3R,4R,1'S)-(-)-Methyl 3-(1'-acetylamino-2'-propyl)pentyl-4-tert-butoxycarbonyl- amino-2-hydroxycyclopentan-1-carboxylate (29, Scheme 2)

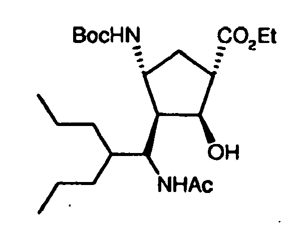

It was prepared from 17 (15 g) using the same procedure as for compound 26.

### Example 30

### (1R,2R,3S,4S,1'R)-(+)-Ethyl 3-(1'-acetylamino-2'-ethyl)butyl-4-tert-butoxycarbonylamino-2-hydroxycyclopentan-1-carboxylate (30, Scheme 2)

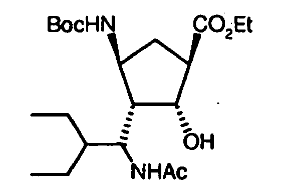

It was prepared from 18 (5.9 g) using the same procedure as for compound 26.

### Examples 31

### (±)-Methyl t-3-(1'-acetylamino-2'-ethyl)butyl-c-4-tert-butoxycarbonylamino-t-2-hydroxy cyclopentan-r-1-carboxylate (31, Isomer-A at C-1', Scheme 2)

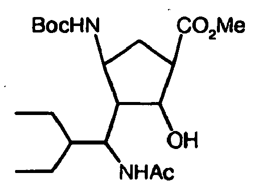

It was prepared from 19 (2.6 g) using the same procedure as for compound 26.

### Example 32

### (+)-Methyl t-3-(1'-acetylamino-2'-propyl)pentyl-c-4-tert-butoxycarbonylamino-t-2-hydroxycyclopentan-r-1-carboxylate (32, Isomer-A at C-1', Scheme 2)

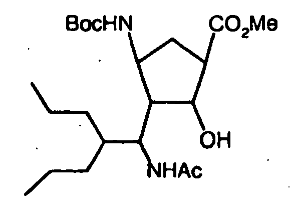

It was prepared from 20 (6.2 g) using the same procedure as for compound 26.

### Example 33

### (±)-Methyl t-3-(1'-acetylamino-3'-ethyl)pentyl-c-4-tert-butoxycarbonylamino-t-2-acetyloxycyclopentan-r-1-carboxylate (33, Isomer-A at C-1', Scheme 2)

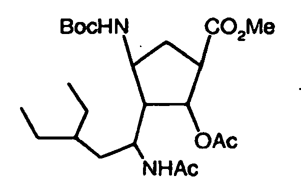

It was prepared from 21 (2.4 g) using the same procedure as for compound 26, except that during acetylation, excess of acetic anhydride (2.5 equivalent) and triethylamine (2.5 equivalent) were used.

### Example 34

### (±)-Methyl t-3-(1'-acetylamino-n-butyl)-c-4-tert-butoxycarbonylamino-t-2-acetyloxycyclopentan-r-1-carboxylate (34, Isomer-A at C-1', Scheme 2)

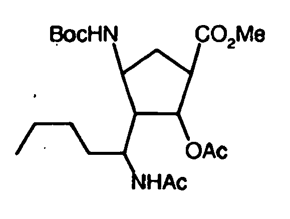

It was prepared from 22 (0.45 g) using the same procedure as for compound 26, except that during acetylation, excess of acetic anhydride (2.5 equivalent) and triethylamine (2.5 equivalent) were used.

### Example 35

### (±)-Ethyl t-3-(1'-acetylamino-2'-ethyl)butyl-c-4-tert-butoxycarbonylamino-t-2-hydroxycyclopentan-r-1-carboxylate (35, Isomer-A at C-1', Scheme 2)

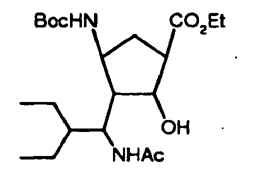

It was prepared from 23 (2.6 g) using the same procedure as for compound 26.

### Example 36

### (±)-Ethyl t-3-(1'-acetylamino-2'-methyl)butyl-c-4-tert-butoxycarbonylamino-t-2-hydroxycyclopentan-r-1-carboxylate (36, Isomer-A at C-1', mixture at C-2', Scheme 2)

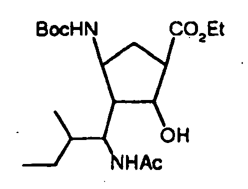

It was prepared from 24 (1.3 g) using the same procedure as for compound 26.

### Example 37

### (±)-Ethyl t-3-(1'-acetylamino-2'-methyl)propyl-c-4-tert-butoxycarbonylamino-t-2-hydroxycyclopentan-r-1-carboxylate (37, Isomer-A at C-1', Scheme 2)

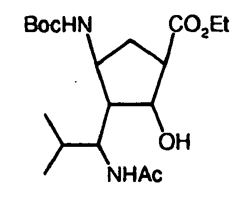

It was prepared from 25 (1.39 g) using the same procedure as for compound 26.

### Example 38

### (1S,2S,3R,4R,1'S)-(-)-Methyl 3-(1'-acetylamino-2'-ethyl)butyl-4-amino-2-hydroxycyclopentan-1-carboxylate Hydrochloride (38, Scheme 2)

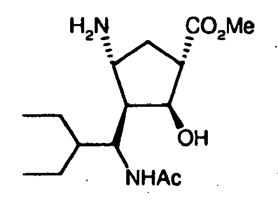

To a mixture of 26 (150 g, 0.375 mol) in ether (800 mL) was added IN HCl in ether (1170 mL, 1.17 mol) and stirred at room temperature for 24 h and then heated at reflux for 2 h. After cooling, the solid was collected by filtration, washed with ether and dried *in vacuo* to give 126 g of 38. This was used as such for the next step.

### Example 39

### (1S,2S,3R,4R,1'S)-(-)-Methyl 3-(1'-acetylamino-2'-propyl)pentyl-4-amino-2-hydroxycyclopentan-1-carboxylate Hydrochloride (39, Scheme 2)

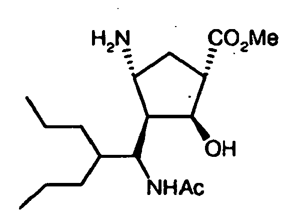

It was prepared from 27 (10.4 g) using the same procedure as for compound 38.

### Example 40

### (1S,2S,3R,4R,1'S)-(-)-Ethyl 3-(1'-acetylamino-2'-ethyl)butyl-4-amino-2-hydroxycyclopentan-1-carboxylate Trifluoroacetate (40, Scheme 2)

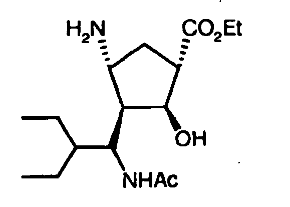

To a mixture of 28 (1.3 g, 3.14 mmol) in dichloromethane (20 mL) was added trifluoroacetic acid (4.0 mL) and stirred at room temperature for 6 h. It was concentrated and co-evaporated 2 times with dichloromethane and 2 times with ether. The residue was dried *in vacuo* to give 40, which was used as such for the next step.

### Example 41

### (1R,2R,3S,4S,1'R)-(+)-Ethyl 3-(1'-acetylamino-2'-ethyl)butyl-4-amino-2-hydroxycyclopentan-1-carboxylate Trifluoroacetate (41, Scheme 2)

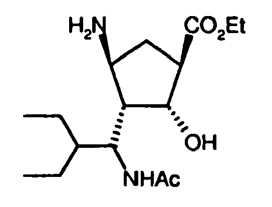

It was prepared from 30 (0.6 g) using the same procedure as for compound 40.

### Example 42

### (±)-Methyl t-3-(1'-acetylamino-2'-ethyl)butyl-c-4-amino-t-2-hydroxycyclopentan-r-1-carboxylate Trifluoroacetate (42, Isomer-A at C-1', Scheme 2)

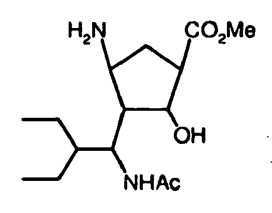

It was prepared from 31 (0.8 g) using the same procedure as for compound 40.

### Example 43

### (±)-Methyl t-3-(1'-acetylamino-2'-propyl)pentyl-c-4-amino-t-2-hydroxycyclopentan-r-1-carboxylate Trifluoroacetate (43, Isomer-A at C-1', Scheme 2)

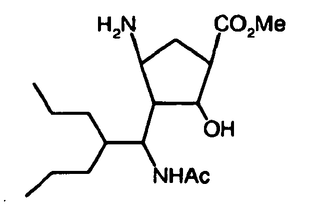

It was prepared from 32 (0.39 g) using the same procedure as for compound 40.

### Example 44

### (±)-Methyl t-3-(1'-acetylamino-3'-ethyl)pentyl-c-4-amino-t-2-acetyloxycyclopentan-r-1-carboxylate Trifluoroacetate (44, Isomer-A at C-1', Scheme 2)

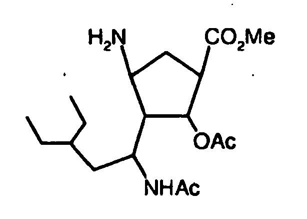

It was prepared from 33 (0.47 g) using the same procedure as for compound 40.

### Example 45

### (±)-Methyl t-3-(1'-acetylamino-n-butyl)-c-4-amino-t-2-acetyloxycyclopentan-r-1-carboxylate Trifluoroacetate (45, Isomer-A at C-1', Scheme 2)

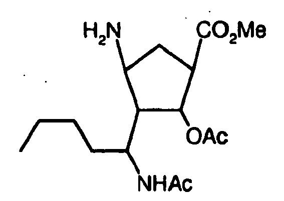

It was prepared from 34 (0.27 g) using the same procedure as for compound 40.

### Example 46

### (±)-Ethyl t-3-(1'-acetylamino-2'-ethyl)butyl-c-4-amino-t-2-hydroxycyclopentan-r-1-carboxylate Trifluoroacetate (46, Isomer-A at C-1', Scheme 2)

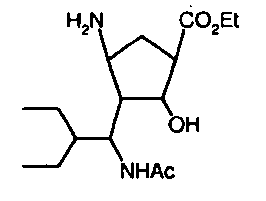

It was prepared from 35 (0.8 g) using the same procedure as for compound 40.

### Examples 47

### (±)-Ethyl t-3-(1'-acetylamino-2'-methyl)butyl-c-4-amino-t-2-hydroxycyclopentan-r-1-carboxylate Trifluoroacetate (47, Isomer-A at C-1', mixture at C-2', Scheme 2)

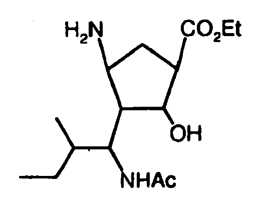

It was prepared from 36 (0.74 g) using the same procedure as for compound 40.

### Example 48

### (±)-Ethyl t-3-(1'-acetylamino-2'-methyl)propyl-c-4-amino-t-2-hydroxycyclopentan-r-1-carboxylate Trifluoroacetate (48, Isomer-A at C-1', Scheme 2)

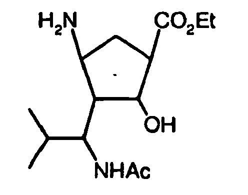

It was prepared from 37 (1.0 g) using the same procedure as for compound 40.

### Examples 49

### (1S,2S,3R,4R,1'S)-(-)-3-(1'-Acetylamino-2'-ethyl)butyl-4-amino-2-hydroxycyclo pentan-1-carboxylic acid (49, Scheme 2)

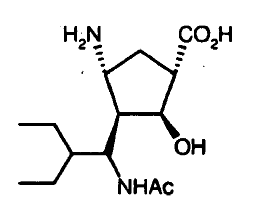

A mixture of 40 (14 mg, 0.0327 mmol) in IN NaOH (0.1 mL) and water (0.2 mL) was stirred at room temperature for 2 h. The reaction mixture was neutralized with 1N HCl and diluted with water to give 32.7 mmolar solution.
MS (ES+): 287.4 (100%, M+1).

### Example 50

### (1R,2R,3S,4S,1'R)-(+)-3-(1'-Acetylamino-2'-ethyl)butyl-4-amino-2-hydroxycyclopentan-1-carboxylic acid (50, Scheme 2)

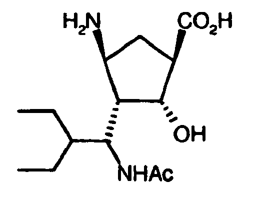

It was prepared from 41 (100 mg, 0.234 mmol) using the same procedure as for compound 49 and was obtained as 93.6 mmolar solution.
MS (ES+): 287.4 (100%, M+1).

### Example 51

### (±)-t-3-(1'-Acetylamino-2'-ethyl)butyl-c-4-amino-t-2-hydroxycyclopentan-r-1-carboxylic acid (51, Isomer-A at C-1', Scheme 2)

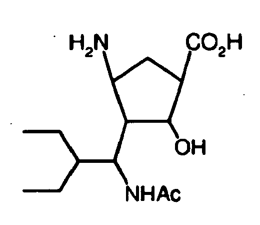

It was prepared from 42 (12.5 mg, 0.030 mmol) using the same procedure as for compound 49 and was obtained as 30.0 mmolar solution.
MS (ES+): 287.4 (100%, M+1).

### Example 52

### (±)-t-3-(1'-Acetylamino-2'-propyl)pentyl-c-4-amino-t-2-hydroxycyclopentan-r-1-carboxylic acid (52, Isomer-A at C-1', Scheme 2)

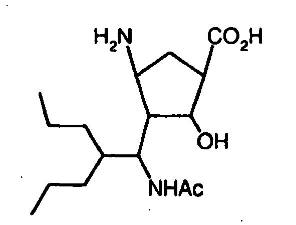

It was prepared from 43 (7.5 mg, 0.017 mmol) using the same procedure as for compound 49 and was obtained as 7.7 mmolar solution.
MS (ES+): 315.5 (100%, M+1).

### Example 53

### (±)-t-3-(1'-Acetylamino-3'-ethyl)pentyl-c-4-amino-t-2-hydroxycyclopentan-r-1-carboxylic acid (53, Isomer-A at C-1', Scheme 2)

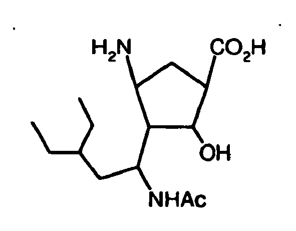

It was prepared from 44 (10.6 mg, 0.0225 mmol) using the same procedure as for compound 49 and was obtained as 21.5 mmolar solution.
MS (ES+): 301.4 (100%, M+1).

### Example 54

### (±)- t-3-(1'-Acetylamino-n-butyl)-c-4-amino-t-2-hydroxycyclopentan-r-1-carboxylic acid (54, Isomer-A at C-1', Scheme 2)

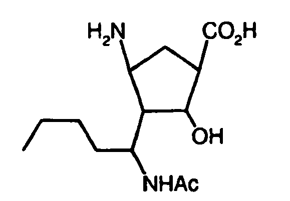

It was prepared from 45 (5.0 mg, 0.011 mmol) using the same procedure as for compound 49 and was obtained as 11.0 mmolar solution.
MS (ES+): 273.0 (100%, M+1).

### Example 55

### (±)-t-3-(1'-Acetylamino-2'-methyl)butyl-c-4-amino-t-2-hydroxycyclopentan-r-1-carboxylic acid (55, Isomer-A at C-1', mixture at C-2', Scheme 2)

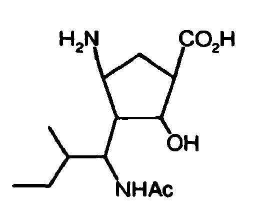

It was prepared from 47 (9.5 mg, 0.0229 mmol) using the same procedure as for compound 49 and was obtained as 9.36 mmolar solution.
MS (ES+): 273.5 (100%, M+1).

### Example 56

### (±)-t-3-(1'-Acetylamino-2'-methyl)propyl-c-4-amino-t-2-hydroxycyclopentan-r-1-carboxylic acid (56, Isomer-A at C-1', Scheme 2)

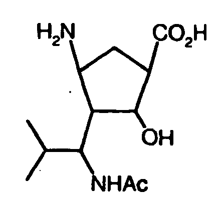

It was prepared from 48 (9.5 mg, 0.0237 mmol) using the same procedure as for compound 49 and was obtained as 12.9 mmolar solution.
MS (ES+): 259.4 (100%, M+1).

### Example 57

### (1S,2S,3R,4R,1'S)-(-)-Ethyl 3-(1'-acetylamino-2'-ethyl)butyl-4-[(tert-butoxycarbonylamino-tert-butoxycarbonylimino)methyl]amino-2-hydroxycyclopentan-1-carboxylate (57, Scheme 3)

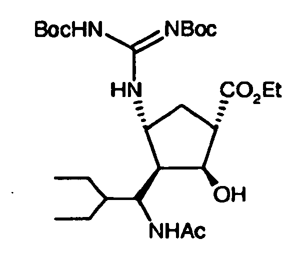

To a mixture of compound 40 (1 mmol) in dry DMF (4 mL) were added Et₃N (3.5 mmol), 1,3-bis(*tert*-butoxycarbonyl)-2-methyl-2-thiopseudourea (1.5 mmol) and HgCl₂ (1.5 mmol). The reaction mixture was stirred at room temperature for 16 h. The mixture was diluted with EtOAc (20 mL) and filtered through Celite. The filtrate was washed with water, brine, and dried (MgSO₄). After filtration, the filtrate was concentrated *in vacuo.* The crude product was purified by flash column chromatography (silica gel, 60-70% EtOAc in hexane) to yield desired compound.

### Example 58

### (±)-Methyl t-3-(1'-acetylamino-2'-ethyl)butyl-c-4-[(tert-butoxycarbonylamino-tert-butoxycarbonylimino)methyl]amino-t-2-hydroxycyclopentan-r-1-carboxylate (58, Isomer-A at C-1', Scheme 3)

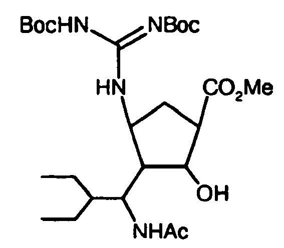

It was prepared from 42 (0.6 g) using the same procedure as for compound 57.

### Example 59

### (±)-Methyl t-3-(1'-acetylamino-3'-ethyl)pentyl-c-4-[(tert-butoxycarbonylamino-tert-butoxycarbonylimino)methyl]amino-t-2-acetyloxycyclopentan-r-1-carboxylate (59, Isomer-A at C-1', Scheme 3)

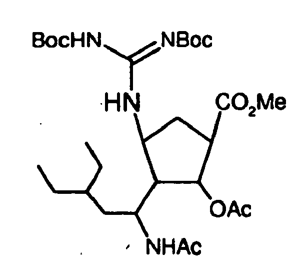

It was prepared from 44 (0.25 g) using the same procedure as for compound 57.

### Example 60

### (±)-Methyl t-3-(1'-acetylamino-n-butyl)-c-4-[(tert-butoxycarbonylamino-tert-butoxycarbonylimino)methyl]amino-t-2-acetyloxycyclopentan-r-1-carboxylate (60, Isomer-A at C-1', Scheme 3)

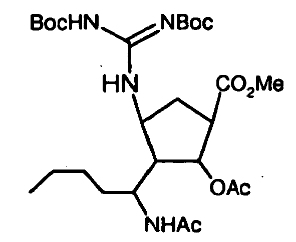

It was prepared from 45 (0.26 g) using the same procedure as for compound 57.

### Example 61

### (1S,2S,3R,4R,1'S)-(-)-Ethyl 3-(1'-acetylamino-2'-ethyl)butyl-4-[(N-tert-butoxycarbonyl-N-methylamino-N'-tert-butoxycarbonylimino)methyl]amino-2-hydroxycyclopentan-1-carboxylate (61, Scheme 3)

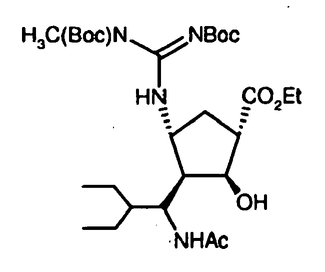

It was prepared from 40 (0.46 g) using the same procedure as for compound 57. The reagent used was 1,3-bis(*tert*-butoxycarbonyl)-N-methyl-2-(2,4-dinitrophenyl)-2-thiopseudourea instead of 1,3-bis(*tert*-butoxycarbonyl)-2-methyl-2-thiopseudourea and HgCl₂ was not required.

### Example 62

### (1S,2S,3R,4R,1'S)-(-)-Ethyl 3-(1'-acetylamino-2'-ethyl)butyl-4-[(amino-imino)methyl]-amino-2-hydroxycyclopentan-1-carboxylate (62, Scheme 3)

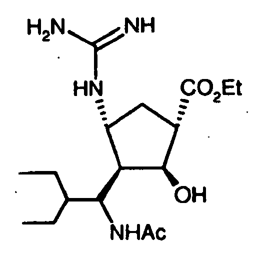

It was prepared from 57 (0.1 g) using the same procedure as for compound 40.

### Example 63

### (±)-Methyl t-3-(1'-acetylamino-2'-ethyl)butyl-c-4-[(amino-imino)methyl]amino-t-2-hydroxycyclopentan-r-1-carboxylate (63, Isomer-A at C-1', Scheme 3)

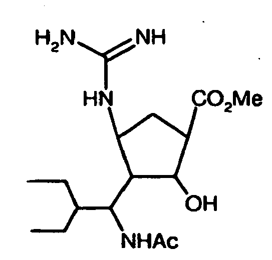

It was prepared from 58 (0.38 g) using the same procedure as for compound 40.

### Example 64

### (±)-Methyl t-3-(1'-acetylamino-3'-ethyl)pentyl-c-4-[(amino-imino)methyl]amino-t-2-acetyloxycyclopentan-r-1-carboxylate (64, Isomer-A at C-1', Scheme 3)

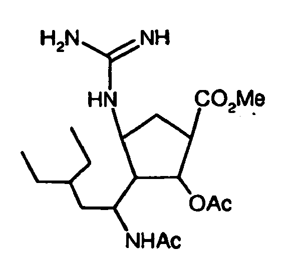

It was prepared from 59 (0.1 g) using the same procedure as for compound 40.

### Example 65

### (±)-Methyl t-3-(1'-acetylamino-n-butyl)-c-4-[(amino-imino)methyl]amino-t-2-acetyloxycyclopentan-r-1-carboxylate (65, Isomer-A at C-1', Scheme 3)

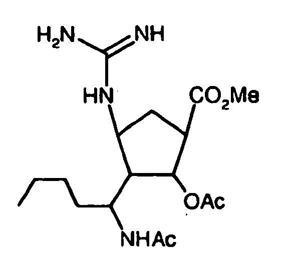

It was prepared from 60 (0.08 g) using the same procedure as for compound 40.

### Example 66

### (1S,2S,3R,4R,1'S)-(-)-Ethyl-3-(1'-acetylamino-2'-ethyl)butyl-4-[(N-methylaminoimino)methyl]amino-2-hydroxycyclopentan-1-carboxylate (66, Scheme 3)

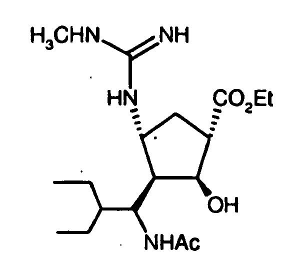

It was prepared from 61 (0.3 g) using the same procedure as for compound 40.

### Example 67

### (1S,2S,3R,4R,1'S)-(-)-3-(1'-Acetylamino-2'-ethyl)butyl-4-[(aminoimino) methyl]amino-2-hydroxycyclopentan-1-carboxylic acid (67, Scheme 3)

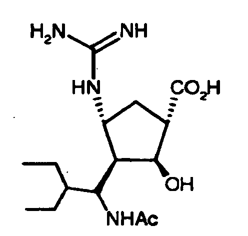

Method-A: It was prepared from 62 (7.8 mg, 0.0166 mmol) using the same procedure as for compound 49 and was obtained as 7.2 mmolar solution.
MS (ES+): 329.5 (M+1).
Method-B: To a mixture of 38 (3.0 g, 8.9 mmol) in DMF (20 mL) was added pyrazole carboxamidine hydrochloride (1.56 g, 10.6 mmol) and di-isopropylethylamine (3.9 mL, 22.4 mmol) and heated at 60 °C for 36 h. Additional amount of pyrazole carboxamidine hydrochloride (0.65 g) and di-isopropyl ethylamine (1 mL) were added and heated at 60 °C for another 12 h. The solvent was evaporated under reduced pressure. To the residue was added IN NaOH (22 mL, 22 mmol) and stirred at room temperature for 5 h The reaction mixture was extracted with ethyl acetate (3x 25 mL) and the aqueous layer was concentrated. The solid was obtained, which was collected by filtration and dried to give 1.22 g (39%) of compound 67.
¹H NMR (D₂O) ¹H NMR (D₂O): δ ppm 0.90 (m, 3 H), 0.95 (m, 3 H), 1.05 (m, 2 H), 1.5 (m, 3 H), 1.8 (m, 1 H), 2.0 (s, 3 H), 2.3 (m, 1 H), 2.55 (m, 1 H), 2.75 (m, 1 H), 3.9 (m, 1 H), 4.4 (m, 2H).

| | | |
|---|---|---|
| Analysis: | Calcd for C₁₅ H₂₈N₄O₄.H₂O: | C, 52.01; H, 8.73; N, 16.17 |
| | Found: | C, 51.64; H, 8.57; N, 16.14 |

### Example 68

### (±)-t-3-(1'-Acetylamino-2'-ethyl)butyl-c-4-[(amino-imino)methyl]amino-t-2-hydroxycyclopentan-r-1-carboxylic acid (68, Isomer-A at C-1', Scheme 3)

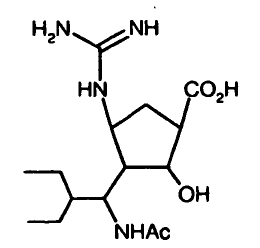

It was prepared from 63 (12.0 mg, 0.0263 mmol) using the same procedure as for compound 49 and was obtained as 26.3 mmolar solution.
MS (ES+): 329.5 (M+1).

### Example 69

### (±)-t-3-(1'-Acetylamino-3'-ethyl)pentyl-c-4-[(amino-imino)methyl]amino-t-2-hydroxycyclopentan-r-1-carboxylic acid (69, Isomer-A at C-1', Scheme 3).

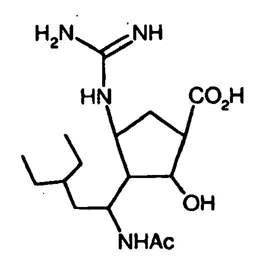

It was prepared from 64 (9.0 mg, 0.0176 mmol) using the same procedure as for compound 49 and was obtained as 17.6 mmolar solution.
MS (ES+): 343.5 (M+1).

### Example 70

### (±)-t-3-(1'-Acetylamino-n-butyl)-c-4-[(amino-imino)methyl]amino-t-2-hydroxycyclopentan-r-1-carboxylic acid (70, Isomer-A at C-1', Scheme 3)

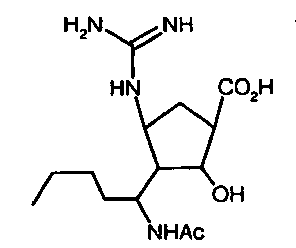

It was prepared from 65 (4.9 mg, 0.010 mmol) using the same procedure as for compound 49 and was obtained as 10.0 mmolar solution.
MS (ES+): 315.0 (M+1).

### Example 71

### (1S,2S,3R,4R,1'S)-(-)-3-(1'-Acetylamino-2'-ethyl)butyl-4-[(N-methylaminoimino)methyl]amino-2-hydroxycyclopentan-1-carboxylic acid (71, Scheme 3)

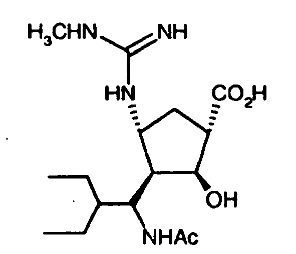

It was prepared from 66 (10.4 mg, 0.0203 mmol) using the same procedure as for compound 49 and was obtained as 20.3 mmolar solution.
MS (ES+): 343.6 (M+1).

### Example 72

### (1S,2S,3R,4R,1'S)-(-)-3-(1'-Acetylamino-2'-propyl)pentyl-4-[(aminoimino)methyl]amino-2-hydroxycyclopentan-1-carboxylic acid (72, Scheme 3)

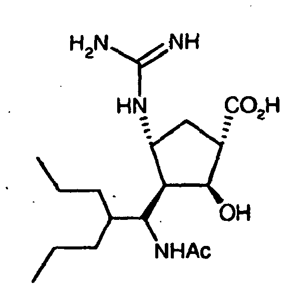

It was prepared from 39 (8.7 g) using the same procedure as for compound 67 (Method-B).

### Biochemistry

The *in vitro* assay is based on the method reported by von Itzstein et al. (EP Application 92309634.6). The neuraminidase from the H1N9 strain of influenza was obtained by the method described by Laver et al. Virology 1984, 137, p. 314-323. Values for the IC 50 were measured via spectrofluorometric technique which uses the flouorogenic substrate, 2' -(4-methylumbelliferyl)-α-D-acetylneuramic acid. This substrate is cleaved by neuraminidase to yield a fluorescent product which can be quantified. The assay mixture contains inhibitors at various concentrations (four to six points) and enzyme in 32.5 mM MES [(2-(N-morpholino) ethanesulfonic acid] buffer, 4mM CaCl₂ at pH = 6.5 (total volume = 80 µL). The reaction is started by the addition of 20 µL of the substrate to a final concentration of 75 µM. After 10 min at 37° C, 150 µl of 0.2M glycine/NaOH (pH = 10.2) is added to 0.1 mL of the reaction mixture to terminate the reaction. A blank is run with the same substrate solution with no enzyme. Fluorescence is read using a spectrafluor Fluorometer (excitation: 360 nm and emission: 450 nm) and readings from substrate blanks were subtracted from the sample readings. The IC₅₀ is calculated by plotting percent inhibition of NA activity versus the inhibitor concentration, and determination of each point is performed in duplicate.

### Biological Data

The following table provides the neuraminidase enzyme inhibition data (IC₅₀ values).

| Compound no. | Flu A | Flu B |
|---|---|---|
| | | |
| 49 | +++ | +++ |
| 51 | +++ | +++ |
| 52 | +++ | +++ |
| 54 | ++ | ++ |
| 55 | +++ | ++ |
| 67 | +++ | +++ |
| 68 | +++ | +++ |
| 69 | +++ | +++ |
| 70 | +++ | +++ |
| 71 | +++ | +++ |
| 72 | +++ | +++ |

| | | |
|---|---|---|
| ++ 1-100 µM; +++ <1 µM. | | |

### Crystallography

Complexes between neuraminidase and inhibition molecules were prepared by transferring H1N9 neuraminidase crystals into 2 mL of the phosphate buffer solution in which the inhibitor has been dissolved. The concentration of the inhibitor compound was adjusted to be 2 mM. The crystal was allowed to equilibrate in the buffer solution for about one day and then removed from the solution and mounted in a glass capillary for X-ray diffraction data collection. All X-ray intensity measurements were recorded with a Siemens X-100 multiwire area detector on a Rigaku RU-300 rotating anode generator operating at 100 mA and 50 KV and a copper anode. The crystal to detector distance was160 mm and the detector was offset 2.2 Å data. Intensity data were measured on 0.1 oscillation frames at 240 s of exposure per fame. Each crystal yielded 600-700 frames of data before radiation damage to the crystals prevented further data collection.

The intensity data were processed using the XENGEN package of programs. The integrated intensities were scaled and merged to produce a final data set containing only unique reflections. All refinement was carried out using the program XPLOR. The starting model for refinement was the 2.0 Å refined native N9 structure. Difference Fourier maps to 2.2 Å were calculated using the calculated phases from the refined model. Analysis of the electron density maps was performed on a Silicon Graphics Indigo Extreme 2 computer graphics workstation using the graphics program QUANTA. Idealized models for the inhibitor molecules were manually fitted to the difference electron density. These inhibitor models were later included in the XPLOR refinement.

### Dosage and formulation

The antiviral compounds of this invention can be administered as treatment for viral infections by any means that produce contact of the active agent's site for action with the viral neuraminidase in the body of a human, mammal, bird, or other animal. They can be administered by a conventional means available for use in conjunction with pharmaceuticals, either as individual therapeutic agents or in a combination of therapeutic agents. They can be administered alone, but generally administered with a pharmaceutical carrier selected on the basis of the chosen route of administration and standard pharmaceutical practice.

The dosage administered will, of course, vary depending upon known factors, such as the pharmacodynamic characteristics of the particular agent and its mode and route of administration; the age, health and weight of the recipient; the nature and extent of the symptoms, the kind of concurrent treatment; the frequency of treatment; and the effect desire. A daily dosage of active ingredient can be expected to be about 0.001 to 1000 milligram (mg) per kilogram (kg) of body weight, with the preferred dose being 0.1 to about 30 mg/kg.

Dosage forms (compositions sutiable for administration) contain from about 1 mg to about 500 mg of active ingredient per unit. In these pharmaceutical compositions, the active ingredient will ordinarily be present in an amount of about 0.5-95% weight based on the total weight of the composition.

The active ingredient can be administered orally in solid dosage forms, such as capsules, tablets, and powders, or in liquid dosage forms, such as elixirs, syrups and suspensions. It can also be administered parenterally, in sterile liquid dosage forms. The active ingredient can also be administered intranasally (nose drops) or by inhalation of a drug powder mist. Other dosage forms are potentially possible such as administration transdermally, via a patch mechanism or ointment.

Gelatin capsules contain the active ingredient and powdered carriers, such as lactose, starch, cellulose derivatives, biocompatible polymers, magnesium stearate, stearic acid, and the like. Similar diluents can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric coated for selective disintegration in the gastrointestinal tract.

Liquid dosage forms for oral administration can contain coloring and flavoring to increase patient acceptance. They may also contain buffering agents, surfactants and preservatives. Liquid oral products can be developed to have sustained-release properties. They may also contain cyclodextrin derivatives to enhance the solubility of the active ingredient and to promote its oral uptake.

In general, water, a suitable oil, saline, aqueous dextrose (glucose), and related sugar solutions and glycol such as propylene glycol or polyethylene glycol are suitable carriers for parental solutions. Solutions for parenteral administration preferably contain a water-soluble salt of the active ingredient, suitable stabilizing agents, and, if necessary, buffering agents. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propylparaben, and chlorobutanol.

Suitable pharmaceutical carriers are described in Remington's Pharmaceutical Sciences, Mack Publishing Company and in the Handbook of Pharmaceuticals Excipients, American Pharmaceutical Association, both standard reference texts in this field.

Useful pharmaceutical dosage forms for administration of the compounds according to the present invention can be illustrated as follows:

### Hard Shell Capsules

A large number of unit capsules are prepared by filling standard two-piece hard gelatin capsules each with 100 mg of powdered active ingredient, 150 mg of lactose, 50 mg of cellulose, and 6 mg of magnesium stearate.

### Soft Gelatin Capsules

A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

### Tablets

A large number of tablets are prepared by conventional procedures so that the dosage unit was 100 mg of active ingredient, 0.2 mg of colloidal silicon dioxide, 5 mg of magnesium stearate, 275 mg of microcrystalline cellulose, 11 mg of starch, and 98.8 mg of lactose. Appropriate aqueous and non-aqueous coatings may be applied to increase palatability, improve elegance and stability or delay absorption.

### Immediate Release Tablets/Capsules

These are solid oral dosage forms made by conventional and novel processes. These units are taken orally without water for immediate dissolution and delivery of the medication. The active ingredient is mixed in liquid containing ingredient such as sugar, gelatin, pectin, and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

Moreover, the compounds of the present invention can be administered in the form of nose drops, or metered dose and a nasal or buccal inhalers. The drug is delivered from a nasal solution as a fine mist or from a powder as an aerosol.

## Claims

1. A compound represented by the formula: 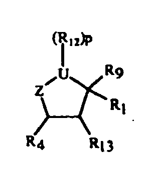 wherein
U is CH and p is 1
Z is -C(R₂)(R₃), -CH-N(R₂)(R₃), C(R₃)[(CH₂)ₙR₂], CH-C(R₃)(R₈)(CH₂)ₙR₂,
C[(CH₂)ₙR₂]-[CH(R₃)(R₈)], or C[(R₃)][CH(CH₂)ₙR₂(R₈)];
R₁ is H, (CH₂)ₙOH, (CH₂)ₙNH₂, (CH₂)ₙNR₁₀R₁₁, (CH₂)ₙOR₁₁, (CH₂)ₙSR₁₁, or (CH₂)ₙ halogen
R₉ is (CH₂)-CO₂H, (CH₂)ₙSO₃H, or (CH₂)ₙPO₃H₂, esters thereof, or salts thereof;
R₂ is NHC(O)Rs, NHC(S)R₅, NHSO₂R₅, C(O)NHR₅, SO₂NHR₅, CH₂S(O)R₅, or CH₂SO₂R₅;
R₃ and R₈ individually is H, (CH₂)ₙC(O)R₁₀, (CH₂)ₙCO₂R₁₀, (CH₂)ₙOR₁₀ CH(OR₁₀)CH(R₁₀)ₘ, C(O)N(R₁₀)ₘ, C(O)N(OR₁₀)R₁₀, (CH₂)ₙN(R₁₀)ₘ, CH(R₁₀)ₘ when m is 2, (CH₂)ₙ(R₁₀)ₘ, CH₂CH(OR₁₀)CH₂OR₁₀, CH(OR₁₀)CH(OR₁₀)CH₂OR₁₀, CH₂OR₁₀, CH(OR₁₀)CH₂NHR₁₀, CH₂CH(OR₁₀)CH₂NHR₁₀, CH(OR₁₀)CH(OR₁₀)CH₂NHR₁₀, C(=NR₁₀)N(R₁₀)ₘ, NHR₁₀, or NHC(=NR₁₀)N(R₁₀)ₘ,
R₄ is (CH₂)ₙOH, (CH₂)ₙOR₁₁, (CH₂)ₙOC(O)R₁₁, (CH₂)ₙNHC(=NR₁₁)NHR₁₁, (CH₂)ₙNR₁₀R₁₁, (CH₂)ₙNH₂, (CH₂)ₙC(=NH)(NH₂), (CH₂)ₙNHC(=NR₁₁)NH₂, (CH₂)ₙNHC(=NR₇)NH₂, (CH₂)ₙCN, (CH₂)ₙN₃, C(=NH)NH₂, C(=NR₇)NH₂, or C(=NR₁₁)NH₂
R₅ is H, lower alkyl having 1 to 8 carbon atoms, cyclo alkyl, halogen substituted alkyl, aryl, substituted aryl or CF₃;
R₇ is H, (CH₂)ₙOH, (CH₂)ₙCN, (CH₂)ₙNH₂, or (CH₂)ₙNO₂;
R₁₀ is H, lower alkyl having 1 to 8 carbon atoms, lower alkenyl having 2 to 8 carbon atoms, branched alkyl, cyclic alkyl, (CH₂)ₙ aromatic, (CH₂)₆ substituted aromatic, or when m is 2 both R₁₀ groups can also be interconnected to form an N substituted heterocyclic ring, or other 5- or 6-membered heterocyclic ring;
R₁₁ is lower alkyl having 1 to 8 carbon atoms, branched alkyl, (CH₂)ₘ aromatic, SO₂R₁₀, C(O)R₁₀ or C(O)OR₁₀;
R₁₂ is (CH₂)ₙOH, R₁₃ is H;
m is 1 or 2;
n is 0-4;
and pharmaceutically acceptable salts thereof.

2. The compound of claim 1, wherein said lower alkyl group contains 1 to 3 carbon atoms; and said lower alkenyl group contains 2 to 3 carbon atoms.

3. The compound of claim 1, wherein said alkyl group R₅, R₁₀ and R₁₁ is selected from the group consisting of methyl, ethyl, propyl, isopropyl, t-butyl, cyclopentyl, and cyclohexyl, the aromatic group is selected from the group consisting of phenyl and alkyl substituted aromatic groups; the substituted cycloalkyl group contains 3-8 carbon atoms in the ring and are substituted with 1 or 2 alkyl groups having 1-6 carbon atoms, hydroxy group or both; and the alkenyl group is selected from the group consisting of vinyl, I-propenyl, allyl, isopropenyl, 2-methyl-2-propenyl and cyclopentenyl.

4. The compound of claim 1, wherein said salt is from an acid selected from the group consisting of hydrochloric, hydrobromic, sulphuric, nitric, perchloric, fumaric, maleic, phosphoric, glycollic, lactic, salicyclic, succinic, toluene-p-sulphonic, tartaric, acetic, citric, methanesulphonic, formic, benzoic, malonic, naphthalene-2-sulphonic, trifluoroacetic and benzenesulphonic acid.

5. The compound of claim 1, wherein said salt is a sodium or ammonium salt when R₉ is (CH₂)nCO₂H, (CH₂)ₙSO₃H and (CH₂)nPO₃H₂.

6. A compound being selected from the group consisting of (1*S*,2*S*,3*R*,4*R*,1'*S*)-(-)-Methyl 3-(1'-acetylamino-2'-ethyl)butyl-4-*tert*-butoxycarbonyl- amino-2-hydroxycyclopentan-1-carboxylate,(1*S*,2*S*,3*R*,4*R*,1'*S*)-(-)-Methyl 3-(1'-acetylamino-2'-propyl)pentyl-4-*tert-*butoxycarbonyl-amino-2-hydroxycyclopentan-1-carboxylate,(1*S*,2*S*,3*R*,4*R*,1'*S*)-(-)-Ethyl 3-(1'-acetylamino-2'-ethyl)butyl-4-*tert-*butoxycarbonyl-amino-2-hydroxycyclopentan-1-carboxylate,(1*S*,2*S*,3*R*,4*R*,1'*S*)-(-)-Methyl 3-(1'-acetylamino-2'-propyl)pentyl-4-*tert*-butoxycarbonyl- amino-2-hydroxycyclopentan-1-carboxylate, (1*R*,2*R*,3*S*,4*S*,1'*R*)-(+)-Ethyl 3-(1'-acetylamino-2'-ethyl)butyl-4-*tert*-butoxycarbonyl-amino-2-hydroxycyclopentan-1-carboxylate,(±)-Methyl *t*-3-(1'-acetylamino-2'-ethyl)butyl-*c*-4-*tert*-butoxycarbonylamino-*t*-2-hydroxy cyclopentan-*r*-1-carboxylate, (±)-Methyl *t*-3-(1'-acetylamino-2'-propyl)pentyl-*c*-4-*tert-*butoxycarbonylamino-*t*-2-hydroxycyclopentan-*r*-1-carboxylate, (±)-Methyl *t*-3-(1'-acetylamino-3'-ethyl)pentyl-*c*-4-*tert-*butoxycarbonylamino-*t*-2-acetyloxycyclopentan-*r-*1-carboxylate, (±)-Methyl *t*-3-(1'-acetylamino-n-butyl)-*c*-4-*tert*-butoxycarbonylamino-*t*-2-acetyloxy- cyclopentan-*r*-1-carboxylate, (±)-Ethyl *t*-3-(1'-acetylamino-2'-ethyl)butyl-*c*-4-*tert*-butoxycarbonylamino-*t*-2-hydroxycyclopentan-*r*-1-carboxylate, (±)-Ethyl *t*-3-(1'-acetylamino-2'-methyl)butyl-*c*-4-*tert*-butoxycarbonylamino-*t*-2-hydroxycyclopentan-*r*-1-carboxylate, (±)-Ethyl *t*-3-(1'-acetylamino-2'-methyl)propyl-*c-*4-*tert*-butoxycarbonylamino-*t*-2-hydroxycyclopentan-*r*-1-carboxylate,(1*S*,2*S*,3*R*,4*R*,1'*S*)-(-)-Methyl 3-(1'-acetylamino-2'-ethyl)butyl-4-amino-2-hydroxy-cyclopentan-1-carboxylate hydrochloride, (1*S*,2*S*,3*R*,4*R*,1'*S*)-(-)-Methyl 3-(1'-acetylamino-2'-propyl)pentyl-4-amino-2-hydroxycyclopentan-1-carboxylate hydrochloride,(1*S,*2*S*,3*R*,4R,1'*S*)-(-)-Ethyl 3-(1'-acetylamino-2'-ethyl)butyl-4-amino-2-hydroxycyclo-pentan-1-carboxylate trifluoroacetate,1*R*,2*R*,3*S*,4*S*,1'*R*)-(+)-Ethyl 3-(1'-acetylamino-2'-ethyl)butyl-4-amino-2-hydroxy- cyclopentan-1-carboxylate trifluoroacetate, (±)-Methyl *t*-3-(1'-acetylamino-2'-ethyl)butyl-*c*-4-amino-*t*-2-hydroxycyclopentan-*r*-1-carboxylate trifluoroacetate, (±)-Methyl *t*-3-(1'-acetylamino-2'-propyl)pentyl-*c*-4-amino-*t*-2-hydroxycyclopentan-*r*-1-carboxylate trifluoroacetate, (±)-Methyl *t*-3-(1'-acetylamino-3'-ethyl)pentyl-*c*-4-amino-*t*-2-acetyloxycyclopentan-*r-*1-carboxylate trifluoroacetate, (±)-Methyl *t*-3-(1'-acetylamino-n-butyl)-*c*-4-amino-*t*-2-acetyloxycyclopentan-*r*-1-carboxylate trifluoroacetatc,(±)-Ethyl *t*-3-(1'-acetylamino-2'-ethyl)butyl-*c*-4-amino-*t*-2-hydroxycyclopentan-*r*-1-carboxylate trifluoroacetate, (±)-Ethyl *t*-3-(1'-acetylamino-2'-methyl)butyl-*c*-4-amino-*t*-2-hydroxycyclopentan-*r*-1-carboxylate trifluoroacetate, (±)-Ethyl *t*-3-(1'-acetylamino-2'-methyl)propyl-*c*-4-amino-*t*-2-hydroxycyclopentan-*r*-1-carboxylate trifluoroacetate, (1*S*,2*S*,3*R*,4*R*,1'*S*)-(-)-3-(1'-Acetylamino-2'-ethyl)butyl-4-amino-2-hydroxycyclo pentan-1-carboxylic acid, (1*R*,2*R*,3*S*,4*S*,1'R)-(+)-3-(1'-Acetylamino-2'-ethyl)butyl-4-amino-2-hydroxycyctopentan-1-carboxylic acid, (±)-*t*-3-(1'-Acetylamino-2'-ethyl)butyl-*c*-4-amino-*t*-2-hydroxycyclopentan-*r*-1-carboxylic acid, (±)-*t*-3-(1'-Acetylamino-2'-propyl)pentyl-*c*-4-amino-*t*-2-hydroxycyclopentan-*r*-1-carboxylic acid, (±)-*t*-3-(1'-Acetylamino-3'-ethyl)pentyl-*c*-4-amino-*t*-2-hydroxycyclopentan-*r*-1-carboxylic acid, (±)- *t*-3-(1'-Acetylamino-n-butyl)-*c*-4-amino-*t*-2-hydroxycyclopentan-*r*-1-carboxylic acid, (±)-*t*-3-(1'-Acetylamino-2'-methyl)butyl-*c*-4-amino-*t*-2-hydroxycyclopentan-*r*-1-carboxylic acid, (±)-*t*-3-(1'-Acetylamino-2'-methyl)propyl-*c*-4-amino-*t*-2-hydroxycyclopentan-*r*-1-carboxylic acid, (1*S*,2*S*,3*R*,4*R*,1'*S*)-(-)-Ethyl 3-(1'-acetylamino-2'-ethyl)butyl-4-{(*tert-*butoxycarbo- nyl-amino-*tert*-butoxycarbonylimino)methyl}amino-2-hydroxycyclopentan-1-carboxylate, (±)-Methyl *t*-3-(1'-acetylamino-2'-ethyl)butyl-*c*-4-{(*tert*-butoxycarbonylamino-*tert*-butoxycarbonylimino)methyl}amino-*t-*2-hydroxycyclopentan-*r*-1-carboxylate, (±)-Methyl *t-*3-(1'-acetylamino-3'-ethyl)pentyl-3'-4-{(*tert*-butoxycarbonylamino-*tert*-butoxycarbonylimino)methyl}amino-*t*-2-acetyloxycyclopentan-*r*-1-carboxylate, (±)-Methyl *t*-3-(1'-acetylamino-n-butyl)-*c*-4-{(*tert*-butoxycarbonylamino-*tert*-butoxycarbonylimino)methyl}amino-*t*-2-acetyloxycyclopentan-*r*-1-carboxylate, (1*S*,2*S*,3*R*,4*R*,1'*S*)-(-)-Ethyl 3-(1'-acetylamino-2'-ethyl)butyl-4-{(N-*tert*-butoxycarbonyl-N-methylamino-N'-*tert-*butoxycarbonylimino)methyl}amino-2-hydroxycyclopentan-1-carboxylate, (1*S*,2*S*,3*R*,4*R*,1'*S*)-(-)-Ethyl 3-(1'-acetylamino-2'-ethyl)butyl-4-{(aminoimino)methyl}- amino-2-hydroxycyclopentan-1-carboxylate, (±)-Methyl *t*-3-(1'-acetylamino-2'-ethyl)butyl-*c*-4-{(amino-imino)methyl}amino-*t*-2-hydroxycyclopentan-*r*-1-carboxylate, (±)-Methyl *t*-3-(1'-acetylamino-3'-ethyl)pentyl-*c*-4-{(aminoimino)methyl}amino-*t*-2-acetyloxycyclopentan-*r*-1-carboxylate, (±)-Methyl *t*-3-(1'-acetylamino-n-butyl)-*c*-4- {(amino-imino)methyl}amino-*t*-2-acetyloxycyclopentan-*r*-1-carboxylate, (1*S*,2*S*,3*R*,4*R*,1'*S*)-(-)-Ethyl-3-(1'-acetylamino-2'-ethyl)butyl-4-{(N-methylamino-imino)methyl}amino-2-hydroxycyclopentan-1-carboxylate, (1*S*,2*S*,3*R*,4*R*,1'*S*)-(-)-3-(1'-Acetylamino-2'-ethyl)butyl-4-{(aminoimino) methyl}amino-2-hydroxycyclopentan-1-carboxylic acid, (±)-*t*-3-(1'-Acetylamino-2'-ethyl)butyl-*c*-4-{(amino-imino)methyl}amino-*t*-2-hydroxycyclopentan-*r*-1-carboxylic acid, (±)-*t*-3-(1'-Acetylamino-3'-ethyl)pentyl-*c*-4-{(amino-imino)methyl}amino-*t*-2-hydroxycyclopentan-*r*-1-carboxylic acid, (±)-*t*-3-(1'-Acetylamino-n-butyl)-*c*-4-{(amino-imino)methyl}amino-*t*-2-hydroxycyclopentan-*r*-1-carboxylic acid, (1*S*,2*S*,3*R*,4*R*,1'*S*)-(-)-3-(1'-Acetylamino-2'-ethyl)butyl-4-{(N-methylaminoimino)methyl}amino-2-hydroxycyclopentan-1-carboxylic acid, (1*S*,2*S*,3*R*,4*R*,1'*S*)-(-)-3-(1'-Acetylamino-2'-propyl)pentyl-4-{(amino-imino)methyl} amino-2-hydroxycyclopentan-1-carboxylic acid and pharmaceutically acceptable salts thereof,

7. The compound of claim 6 being selected from the group consisting of (1*S*,2*S*,3*R*,4*R*,1'*S*)-(-)-3-(1'-Acetylamino-2'-ethyl)butyl-4-amino-2-hydroxycyclo pentan-1-carboxylic acid, (±)-*t*-3-(1'-Acetylamino-2'-ethyl)butyl-*c*-4-amino-*t*-2-hydroxycyclopentan-*r*-1-carboxylic acid, (±)-*t*-3-(1'-Acetylamino-2'-propyl)pentyl-*c*-4-amino-*t*-2-hydroxycyclopentan-*r*-1-carboxylic acid, (±)- *t*-3-(-1'-Acetylamino-n-butyl)-*c*-4-amino-*t*-2-hydroxycyclopentan-*r*-1-carboxylic acid, (±)-*t*-3-(1'-Acetylamino-2'-methyl)butyl-*c*-4-amino-*t*-2-hydroxycyclopentan-*r*-1-carboxylic acid, (1*S*,2*S*,3*R*,4*R*,1'*S*)-(-)-3-(1'-Acetylamino-2'-ethyl)butyl-4-{(aminoimino) methyl}amino-2-hydroxycyclopentan-1-carboxylic acid,(±)-*t*-3-(1'-Acetylamino-2'-ethyl)butyl-*c*-4-{(amino-imino)methyl}amino-*t*-2-hydroxycyclopentan-*r*-1-carboxylic acid, (±)-*t*-3-(1'-Acetylamino-3'-ethyl)pentyl-*c*-4-{(amino-imino)methyl)amino-*t*-2-hydroxycyclopentan-*r*-1-carboxylic acid, (±)-*t*-3-(1'-Acetylamino-n-butyl)-*c*-4-[(aminoimino)methyl]amino-*t*-2-hydroxycyclopentan-*r*-1-carboxylic acid, (1*S,*2*S,*3*R,*4*R,*1*'S-*(-)-3-(1'-Acetylamino-2'-ethyl)butyl-4-{(N-methylamino-imino)methyl}amino-2-hydroxycyclopentan-1-carboxylic acid, (1*S*,2*S*,3*R*,4*R*,1'*S*)-(-)-3-(1'-Acetylamino-2'-propyl)pentyl-4- {(amino-imino)methyl} amino-2-hydroxycyclopentan-1-carboxylic acid, and pharmaceutically acceptable salts thereof.

8. The compound of claim 6 being (1S,2S,3R,4R,1'S)-(-)-Methyl-3-(1'-acetylamino-2'-ethyl)butyl-4-amino-2-hydroxy-cyclopentan-1-carboxylate, or a pharmaceutically acceptable salt thereof.

9. The compound of claim 6 being (1S,2S,3R,4R,1'S)-(-)-Methyl-3-(1'-acetylamino-2'-propyl)pentyl-4-amino-2-hydroxy-cyclopentan-1-carboxylate, or a pharmaceutically acceptable salt thereof.

10. The compound of claim 6 being (1S,2S,3R,4R,1'S)-(-)-Ethyl-3-(1'-acetylamino-2'-ethyl)butyl-4-amino-2-hydroxy-cyclopentan-1-carboxylate, or a pharmaceutically acceptable salt thereof.

11. The compound of claim 6 being (1R,2R,3S,4S,1'R)-(+)-Ethyl-3-(1'-acetylamino-2'-ethyl)butyl-4-amino-2-hydroxy-cyclopentan-1-carboxylate, or a pharmaceutically acceptable salt thereof.

12. The compound of claim 6 being (1S,2S,3R,4R,1'S)-(-)-3-(1'-Acetylamino-2'-ethyl)butyl-4-amino-2-hydroxy-cyclopentan-1-carboxylic acid, or a pharmaceutically acceptable salt thereof.

13. The compound of claim 6 being (1R,2R,35,45,1'R)-(+)-3-(1'-Acetylamino-2'-ethyl)butyl-4-amino-2-hydroxy-cyclopentan-1-carboxylic acid, or a pharmaceutically acceptable salt thereof.

14. The compound of claim 6 being (1S,2S,3R,4R,1'S)-(-)-Ethyl-3-(1'-acetylamino-2'-ethyl)butyl-4-[(aminoimino)-methyl]amino-2-hydroxycyclopentan-1-carboxylate, or a pharmaceutically acceptable salt thereof.

15. The compound of claim 6 being (1S,2S,3R,4R,1'S)-(-)-Ethyl-3-(1'-acetylamino-2'-ethyl)butyl-4-[(N-methylamino-imino)methyl]amino-2-hydroxycyclopentan-1-carboxylate, or a pharmaceutically acceptable salt thereof.

16. The compound of claim 7 being (1S,2S,3S,4R,1'S)-(-)-3-(1'-Acetylamino-2'-ethyl)butyl-4-[(aminoimino)methyl]-amino-2-hydroxycyclopentan-1-carboxylic acid, or a pharmaceutically acceptable salt thereof.

17. The compound of claim 7 being (1S,2S,3R,4R,1'S)-(-)-3-(1'-Acetylamino-2'-ethyl)butyl-4-[(N-methylamino-imino)methyl]amino-2-hydroxycyclopentan-1-carboxylic acid, or a pharmaceutically acceptable salt, thereof.

18. The compound of claim 7 being (1S,2S,3R,4R,1'S)-(-)-3-(1'-Acetylamino-2'-propyl)pentyl-4-[(aminoimino)-methyl]amino-2-hydroxycyclopentan-1-carboxylic acid, or a pharmaceutically acceptable salt thereof.

19. The compound of claim 1 being (1S,2S,3R,4R,1'S)-(-)-3-(1'-Acetylamino-2'-propyl) pentyl-4-amino-2-hydroxy-cyclopentan-1-carboxylic acid; or a pharmaceutically acceptable salt thereof.

20. The compound of claim 6 being (1R,2R,3S,4S,1'R)-(+)-3-(1'-Acetylamino-2'-propyl)pentyl-4-amino-2-hydroxy-cyclopentan-1-carboxylic acid, or a pharmaceutically acceptable salt thereof.

21. A composition for inhibiting influenza virus neuraminidase, comprising a pharmaceutically acceptable carrier and an amount effective for inhibiting influenza virus neuraminidase of a compound according to claim 1.

22. A composition for treating influenza virus infection, comprising a pharmaceutically acceptable carrier and an amount effective for inhibiting influenza virus neuraminidase of a compound according to claim 1.

## Patentansprüche

1. Verbindung, dargestellt durch die Formel: 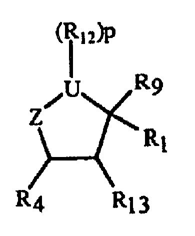 wobei
**U** CH darstellt, und p den Wert 1 annimmt;
**Z** C(R₂)(R₃), CH-N(R₂)(R₃), C(R₃)[(CH₂)ₙR₂], CH-C(R₃)(R₈)(CH₂)ₙR₂, C[(CH₂)ₙR_{2]}-[CH(R₃)(R₈)], oder C[(R₃)][CH(CH₂)ₙR₂(R₈)] darstellt;
**R₁** H, (CH₂)ₙOH, (CH₂)ₙNH₂, (CH₂)ₙNR₁₀R₁₁, (CH₂)ₙOR₁₁, (CH₂)ₙSR₁₁, oder (CH₂)ₙ-Halogen darstellt;
**R₉** (CH₂)ₙCO₂H, (CH₂)ₙSO₃H, oder (CH₂)ₙPO₃H₂, Ester derselben, oder Salze derselben darstellt;
**R₂** NHC(O)R₅, NHC(S)R₅, NHSO₂R₅, C(O)NHR₅, SO₂NHR₅, CH₂S(O)R₅, oder CH₂SO₂R₅ darstellt;
**R₃** und **R₈** jeweils unabhängig H, (CH₂)ₙC(O)R₁₀, (CH₂)ₙCO₂R₁₀, (CH₂)ₙOR₁₀, CH(OR₁₀)CH(R₁₀)ₘ, C(O)N(R₁₀)ₘ, C(O)N(OR₁₀)R₁₀, (CH₂)ₙN(R₁₀)ₘ, CH(R₁₀)ₘ, falls m den Wert 2 annimmt, (CH₂)ₙ(R₁₀)ₘ, CH₂CH(OR₁₀)CH₂OR₁₀, CH(OR₁₀)-CH(OR₁₀)CH₂OR₁₀, CH₂OR₁₀, CH(OR₁₀)CH₂NHR₁₀, CH₂CH(OR₁₀)CH₂NHR₁₀, CH(OR₁₀)CH(OR₁₀)CH₂NHR₁₀, C(=NR₁₀)N(R₁₀)ₘ, NHR₁₀, oder NHC(=NR₁₀)N(R₁₀)ₘ, darstellen;
**R₄** (CH₂)ₙOH, (CH₂)ₙOR₁₁, (CH₂)nOC(O)R₁₁, (CH₂)ₙNHC(=NR₁₁)NHR₁₁, (CH₂)ₙNR₁₀R₁₁, (CH₂)ₙNH₂, (CH₂)nC(=NH)(NH₂), (CH₂)ₙNHC(=NR₁₁)NH₂, (CH₂)ₙNHC(=NR₇)NH₂, (CH₂)ₙCN, (CH₂)ₙN₃, C(=NH)NH₂, C(=NR₇)NH₂, oder C(=NR₁₁)NH₂ darstellt;
**R₅** H, niederes Alkyl mit 1 bis 8 Kohlenstoffatomen, Cycloalkyl, mit Halogen substituiertes Alkyl, Aryl, substituiertes Aryl, oder CF₃ darstellt;
**R₇** H, (CH₂)ₙOH, (CH₂)ₙCN, (CH₂)ₙNH₂, oder (CH₂)ₙNO₂ darstellt;
**R₁₀** H, niederes Alkyl mit 1 bis 8 Kohlenstoffatomen, niederes Alkenyl mit 2 bis 8 Kohlenstoffatomen, verzweigtes Alkyl, Cycloalkyl, (CH₂)ₙ-aromatischer Rest oder (CH₂)ₙ-substituierter aromatischer Rest darstellt, oder, falls m den Wert 2 annimmt, beide Gruppen R₁₀ auch miteinander verbunden sein können, um einen N-substituierten heterocyclischen Ring oder einen anderen 5- oder 6-gliedrigen heterocyclischen Ring zu bilden;
**R₁₁** niederes Alkyl mit 1 bis 8 Kohlenstoffatomen, verzweigtes Alkyl, (CH₂)ₘ-aromatischer Rest, SO₂R₁₀, C(O)R₁₀, oder C(O)OR₁₀ darstellt;
**R₁₂** (CH₂)ₙOH darstellt;
**R₁₃** H darstellt;
**m** den Wert 1 oder 2 annimmt;
**n** den Wert 0 bis 4 annimmt;
und pharmazeutisch akzeptable Salze derselben.

2. Verbindung nach Anspruch 1, wobei die niedere Alkylgruppe 1 bis 3 Kohlenstoffatome enthält; und die niedere Alkenylgruppe 2 bis 3 Kohlenstoffatome enthält.

3. Verbindung nach Anspruch 1, wobei die Alkylgruppe R₅, R₁₀ und R₁₁ aus der Gruppe ausgewählt wird, die aus Methyl, Ethyl, Propyl, Isopropyl, *tert*-Butyl, Cyclopentyl und Cyclohexyl besteht; wobei die aromatische Gruppe aus der Gruppe ausgewählt wird, die aus Phenyl und alkylsubstituierten aromatischen Gruppen besteht; wobei die substituierte Cycloalkyl-Gruppe 3 bis 8 Kohlenstoffatome im Ring enthält und mit 1 oder 2 Alkylgruppen substituiert ist, die 1 bis 6 Kohlenstoffatome, Hydroxy-Gruppen oder beide Merkmale aufweisen; und wobei die Alkenyl-Gruppe aus der Gruppe ausgewählt wird, die aus Vinyl, 1-Propenyl, Allyl, Isopropenyl, 2-Methyl-2-propenyl und Cyclopentenyl besteht.

4. Verbindung nach Anspruch 1, wobei das Salz von einer Säure stammt, die aus einer Gruppe ausgewählt wird, welche besteht aus: Chlorwasserstoffsäure, Bromwasserstoffsäure, Schwefelsäure, Salpetersäure, Perchlorsäure, Fumarsäure, Maleinsäure, Phosphorsäure, Glykolsäure, Milchsäure, Salicylsäure, Bernsteinsäure, p-Toluolsulfonsäure, Weinsäure, Essigsäure, Zitronensäure, Methansulfonsäure, Ameisensäure, Benzoesäure, Malonsäure, Naphthalin-2-sulfonsäure, Trifluoressigsäure und Benzolsulfonsäure.

5. Verbindung nach Anspruch 1, wobei das Salz ein Natrium- oder Ammoniumsalz darstellt, wenn der Rest R₉ (CH₂)ₙCO₂H, (CH₂)ₙSO₃H oder (CH₂)ₙPO₃H₂ darstellt.

6. Verbindung, die aus der Gruppe ausgewählt wird, welche besteht aus:
(*1S*,2*S*,*3R*,*4R*,*1*'*S*)-(-)-3-(1'-Acetylamino-2'-ethyl)butyl-4-*tert*-butoxycarbonylamino-2-hydroxycyclopentan-1-carbonsäuremethylester,
(*1*S,*2S*,*3R,*4*R*,*1*'*S*)-(-)-3-(1'-acetylamino-2'-propyl)pentyl-4-*tert*-butoxycarbonylamino-2-hydroxycyclopentan-1-carbonsäuremethylester,
(*1S*,*2S*,*3R*,*4R*,*1*'*S*)-(-)-3-(1'-Acetylamino-2'-ethyl)butyl-4-*tert*-butoxycarbonylamino-2-hydroxycyclopentan-1-carbonsäureethylester,
(*1S*,*2S*,*3R*,*4R*,*1'S*)-(-)-3-(1'-Acetylamino-2'-propyl)pentyl-4-*tert*-butoxycarbonylamino-2-hydroxycyclopentan-1-carbonsäuremethylester,
(*1R*,*2R*,*3S*,*4S*,*1'R*)-(+)-3-(1'-Acetylamino-2'-ethyl)butyl-4-*tert*-butoxycarbonylamino-2-hydroxycyclopentan-1-carbonsäureethylester,
(±)-*trans*-3-(1'-Acetylamino-2'-ethyl)butyl-*cis*-4-*tert*-butoxycarbonylamino-*trans-*2-hydroxycyclopentan-*rac*-1-carbonsäuremethylester,
(±)-*trans*-3-(1'-Acetylamino-2'-propyl)pentyl-*cis*-4-*tert*-butoxycarbonylamino-*trans*-2-hydroxycyclopentan-*rac*-1-carbonsäuremethylester,
(±)-*trans*-3-(1'-Acetylamino-3'-ethyl)pentyl-*cis*-4-*tert*-butoxycarbonylamino-*trans-*2-acetyloxycyclopentan-*rac*-1-carbonsäuremethylester,
(±)-*trans*-3-(1'-Acetylamino-n-butyl)-*cis*-4-*tert*-butoxycarbonylamino-*trans*-2-acetyloxycyclopentan-*rac*-1-carbonsäuremethylester,
(±)-*trans*-3-(1'-Acetylamino-2'-ethyl)butyl-*cis*-4-*tert*-butoxycarbonylamino-*trans-*2-hydroxycyclopentan-*rac*-1-carbonsäureethylester,
(±)-*trans*-3-(1'-Acetylamino-2'-methyl)butyl-*cis*-4-*tert*-butoxycarbonylamino-*trans*-2-hydroxycyclopentan-*rac*-1-carbonsäureethylester,
(±)-*trans*-3-(1'-Acetylamino-2'-methyl)propyl-*cis*-4-*tert*-butoxycarbonylamino-*trans*-2-hydroxycyclopentan-*rac*-1-carbonsäureethylester,
(*1S*,*2S*,*3R*,*4R*,*1'S*)-(-)-3-(1'-Acetylamino-2'-ethyl)butyl-4-amino-2-hydroxycyclopentan-1-carbonsäuremethylester - Hydrochlorid,
(*1S*,*2S*,*3R*,*4R*,*1'S*)-(-)-3-(1'-Acetylamino-2'-propyl)pentyl-4-amino-2-hydroxycyclopentan-1-carbonsäuremethylester - Hydrochlorid,
(*1S*,*2S*,*3R*,*4R*,*1'S*)-(-)-3-(1'-Acetylamino-2'-ethyl)butyl-4-amino-2-hydroxycyclopentan-1-carbonsäureethylester - Trifluoracetat,
(*1R*,*2R*,*3S*,*4S*,*1'R*)-(+)-3-(1'-Acetylamino-2'-ethyl)butyl-4-amino-2-hydroxycyclopentan-1-carbonsäureethylester - Trifluoracetat,
(±)-*trans*-3-(1'-Acetylamino-2'-ethyl)butyl-*cis*-4-amino-*trans*-2-hydroxycyclopentan-*rac*-1-carbonsäuremethylester - Trifluoracetat,
(±)-*trans*-3-(1'-Acetylamino-2'-propyl)pentyl-*cis*-4-amino-*trans*-2-hydroxycyclopentan-*rac*-1-carbonsäuremethylester - Trifluoracetat,
(±)-*trans*-3-(1'-Acetylamino-3'-ethyl)pentyl-*cis*-4-amino-*trans*-2-acetyloxycyclopentan-*rac*-1-carbonsäuremethylester - Trifluoracetat,
(±)-*trans*-3-(1'-Acetylamino-n-butyl)-*cis*-4-amino-*trans*-2-acetyloxycyclopentan*rac*-1-carbonsäuremethylester - Trifluoracetat,
(±)-*trans*-3-(1'-Acetylamino-2'-ethyl)butyl-*cis*-4-amino-*trans*-2-hydroxycyclopentan-*rac*-1-carbonsäureethylester - Trifluoracetat,
(±)-*trans*-3-(1'-Acetylamino-2'-methyl)butyl-*cis*-4-amino-*trans*-2-hydroxycyclopentan-*rac*-1-carbonsäureethylester - Trifluoracetat,
(±)-*trans*-3-(1'-Acetylamino-2'-methyl)propyl-*cis*-4-amino-*trans*-2-hydroxycyclopentan-*rac*-1-carbonsäureethylester - Trifluoracetat,
(1*S*,2*S*,3*R*,4*R*,1'*S*)-(-)-3-(1'-Acetylamino-2'-ethyl)butyl-4-amino-2-hydroxycyclopentan-1-carbonsäure,
(*1R,2R,3S,4S,1'R*)-(+)-3-(1'-Acetylamino-2'-ethyl)butyl-4-amino-2-hydroxycyclopentan-1-carbonsäure,
(±)-*trans*-3-(1'-Acetylamino-2'-ethyl)butyl-*cis*-4-amino-*trans*-2-hydroxycyclopentan-*rac*-1-carbonsäure,
(±)-*trans*-3-(1'-Acetylamino-2'-propyl)pentyl-*cis*-4-amino-*trans*-2-hydroxycyclopentan-*rac*-1-carbonsäure,
(±)-*trans*-3-(1'-Acetylamino-3'-ethyl)pentyl-*cis*-4-amino-*trans*-2-hydroxycyclopentan-*rac*-1-carbonsäure,
(±)-*trans*-3-(1'-Acetylamino-n-butyl)-*cis*-4-amino-*trans*-2-hydroxycyclopentan-*rac*-1-carbonsäure,
(±)-*trans*-3-(1'-Acetylamino-2'-methyl)butyl-*cis*-4-amino-*trans*-2-hydroxycyclopentan-*rac*-1-carbonsäure,
(±)-*trans*-3-(1'-Acetylamino-2'-methyl)propyl-*cis*-4-amino-*trans*-2-hydroxycyclopentan-*rac*-1-carbonsäure,
(1*S*,2*S*,3*R*,4*R*,1'*S*)-(-)-3-(1'-Acetylamino-2'-ethyl)butyl-4-{(*tert*-butoxycarbonylamino-*tert*-butoxycarbonylimino)methyl}amino-2-hydroxycyclopentan-1-carbonsäureethylester,
(±)-*trans*-3-(1'-Acetylamino-2'-ethyl)butyl-*cis*-4-{(*tert*-butoxycarbonylamino-*tert-*butoxycarbonylimino)methyl}amino-*trans*-2-hydroxycyclopentan-*rac*-1-carbonsäuremethylester,
(±)-*trans*-3-(1'-Acetylamino-3'-ethyl)pentyl-*cis*-4-{(*tert*-butoxycarbonylamino-*tert-*butoxycarbonylimino)methyl}amino-*trans*-2-acetyloxycyclopentan-*rac*-1-carbonsäuremethylester,
(±)-*trans*-3-(1'-Acetylamino-n-butyl)-*cis*-4-{(*tert*-butoxycarbonylamino-*tert-*butoxycarbonylimino)methyl}amino-*trans*-2-acetyloxycyclopentan-*rac*-1-carbonsäuremethylester,
(1*S*,2*S*,3*R*,4*R*,1'*S*)-(-)-3-(1'-Acetylamino-2'-ethyl)butyl-4-{(N-*tert*-butoxycarbonyl-N-methylamino-N'-*tert*-butoxycarbonylimino)methyl}amino-2-hydroxycyclopentan-1-carbonsäureethylester,
(1*S*,2*S*,3*R*,4*R*,1'*S*)-(-)-3-(1'-Acetylamino-2'-ethyl)butyl-4-{(amino-imino)methyl}-amino-2-hydroxycyclopentan-1-carbonsäureethylester,
(±)-*trans*-3-(1'-Acetylamino-2'-ethyl)butyl-*cis*-4-{(amino-imino)methyl}amino-*trans*-2-hydroxycyclopentan-*rac*-1-carbonsäuremethylester,
(±)-*trans*-3-(1'-Acetylamino-3'-ethyl)pentyl-*cis*-4-{(amino-imino)methyl}amino-*trans*-2-acetyloxycyclopentan-*rac*-1-carbonsäuremethylester,
(±)-*trans*-3-(1'-Acetylamino-n-butyl)-*cis*-4-{(amino-imino)methyl}amino-*trans-*2-acetyloxycyclopentan-*rac*-1-carbonsäuremethylester,
(1*S*,2*S*,3*R*,4*R*,1'*S*)-(-)-3-(1'-Acetylamino-2'-ethyl)butyl-4-{(N-methylamino-imino)methyl}amino-2-hydroxycyclopentan-1-carbonsäureethylester,
(1*S*,2*S*,3*R*,4*R*,1'*S*)-(-)-3-(1'-Acetylamino-2'-ethyl)butyl-4-{(amino-imino)methyl}-amino-2-hydroxycyclopentan-1-carbonsäure,
(±)-*trans*-3-(1'-Acetylamino-2'-ethyl)butyl-*cis*-4-{(amino-imino)methyl}amino-*trans*-2-hydroxycyclopentan-*rac*-1-carbonsäure,
(±)-*trans*-3-(1'-Acetylamino-3'-ethyl)pentyl-*cis*-4-{(amino-imino)methyl}amino-*trans*-2-hydroxycyclopentan-*rac*-1-carbonsäure,
(±)-*trans*-3-(1'-Acetylamino-n-butyl)-*cis*-4-{(amino-imino)methyl}amino-*trans-*2-hydroxycyclopentan-*rac*-1-carbonsäure,
(*1S*,*2S*,*3R*,*4R*,*1'S*)-(-)-3-(1'-Acetylamino-2'-ethyl)butyl-4-{(N-methylaminoimino)methyl}amino-2-hydroxycyclopentan-1-carbonsäure,
(*1S*,*2S*,*3R*,*4R*,*1'S*)-(-)-3-(1'-Acetylamino-2'-propyl)pentyl-4-{(amino-imino)-methyl}amino-2-hydroxycyclopentan-1-carbonsäure,
und pharmazeutisch akzeptablen Salzen derselben.

7. Verbindung nach Anspruch 6, wobei die Verbindung aus der Gruppe ausgewählt wird, welche besteht aus:
(*1S*,*2S*,*3R*,*4R*,*1'S*)-(-)-3-(1'-Acetylamino-2'-ethyl)butyl-4-amino-2-hydroxycyclopentan-1-carbonsäure,
(±)-*trans*-3-(1'-Acetylamino-2'-ethyl)butyl-*cis*-4-amino-*trans*-2-hydroxycyclopentan-*rac*-1-carbonsäure,
(±)-*trans*-3-(1'-Acetylamino-2'-propyl)pentyl-*cis*-4-amino-*trans*-2-hydroxycyclopentan-*rac*-1-carbonsäure,
(±)-*trans*-3-(1'-Acetylamino-n-butyl)-*cis*-4-amino-*trans*-2-hydroxycyclopentan-*rac*-1-carbonsäure,
(±)-*trans*-3-(1'-Acetylamino-2'-methyl)butyl-*cis*-4-amino-*trans*-2-hydroxycyclopentan-*rac*-1-carbonsäure,
(*1S*,*2S*,*3R*,*4R*,*1'S*)-(-)-3-(1'-Acetylamino-2'-ethyl)butyl-4-{(amino-imino)methyl}-amino-2-hydroxycyclopentan-1-carbonsäure,
(±)-*trans*-3-(1'-Acetylamino-2'-ethyl)butyl-*cis*-4-{(amino-imino)methyl}amino-*trans*-2-hydroxycyclopentan-*rac*-1-carbonsäure,
(±)-*trans*-3-(1'-Acetylamino-3'-ethyl)pentyl-*cis*-4-{(amino-imino)methyl}amino-*trans*-2-hydroxycyclopentan-*rac*-1-carbonsäure,
(±)-*trans*-3-(1'-Acetylamino-n-butyl)-*cis*-4[(amino-imino)methyl]amino-*trans-*2-hydroxycyclopentan-*rac*-1-carbonsäure,
(*1S*,*2S*,*3R*,*4R*,*1'S*)-(-)-3-(1'-Acetylamino-2'-ethyl)butyl-4-{(N-methylamino-imino)methyl}amino-2-hydroxycyclopentan-1-carbonsäure,
(*1S*,*2S*,*3R*,*4R*,*1'S*)-(-)-3-(1'-Acetylamino-2'-propyl)pentyl-4-{(amino-imino)-methyl}amino-2-hydroxycyclopentan-1-carbonsäure,
und pharmazeutisch akzeptablen Salzen derselben.

8. Verbindung nach Anspruch 6, welche darstellt: (*1S*,*2S*,*3R*,*4R*,*1'S*)-(-)-3-(1'-Acetylamino-2'-ethyl)butyl-4-amino-2-hydroxy-cyclopentan-1-carbonsäuremethylester, oder ein pharmazeutisch akzeptables Salz derselben.

9. Verbindung nach Anspruch 6, welche darstellt: (*1S*,*2S*,*3R*,*4R*,*1'S*)-(-)-3-(1'-Acetylamino-2'-propyl)pentyl-4-amino-2-hydroxy-cyclopentan-1-carbonsäuremethylester, oder ein pharmazeutisch akzeptables Salz derselben.

10. Verbindung nach Anspruch 6, welche darstellt: (*1S*,*2S*,*3R*,*4R*,*1'S*)-(-)-3-(1'-Acetylamino-2'-ethyl)butyl-4-amino-2-hydroxy-cyclopentan-1-carbonsäure-ethylester, oder ein pharmazeutisch akzeptables Salz derselben.

11. Verbindung nach Anspruch 6, welche darstellt: (*1R*,*2R*,*3S*,*4S*,*1'R*)-(+)-3-(1'-Acetylamino-2'-ethyl)butyl-4-amino-2-hydroxy-cyclopentan-1-carbonsäure-ethylester, oder ein pharmazeutisch akzeptables Salz derselben.

12. Verbindung nach Anspruch 6, welche darstellt: (*1S*,*2S*,*3R*,*4R*,*1'S*)-(-)-3-(1'-Acetylamino-2'-ethyl)butyl-4-amino-2-hydroxycyclopentan-1-carbonsäure, oder ein pharmazeutisch akzeptables Salz derselben.

13. Verbindung nach Anspruch 6, welche darstellt: (*1R*,*2R*,*3S*,*4S*,*1'R*)-(+)-3-(1'-Acetylamino-2'-ethyl)butyl-4-amino-2-hydroxycyclopentan-1-carbonsäure, oder ein pharmazeutisch akzeptables Salz derselben.

14. Verbindung nach Anspruch 6, welche darstellt: (*1S*,*2S*,*3R*,*4R*,*1'S*)-(-)-3-(1'-Acetylamino-2'-ethyl)butyl-4-[(amino-imino)methyl]amino-2-hydroxy-cyclopentan-1-carbonsäureethylester, oder ein pharmazeutisch akzeptables Salz derselben.

15. Verbindung nach Anspruch 6, welche darstellt: (*1S*,*2S*,*3R*,*4R*,*1'S*)-(-)-3-(1'-Acetylamino-2'-ethyl)butyl-4-[(N-methylamino-imino)methyl]amino-2-hydroxycyclopentan-1-carbonsäureethylester, oder ein pharmazeutisch akzeptables Salz derselben.

16. Verbindung nach Anspruch 7, welche darstellt: (*1S*,*2S*,*3R*,*4R*,*1'S*)-(-)-3-(1'-Acetylamino-2'-ethyl)butyl-4-[(amino-imino)methyl]amino-2-hydroxycyclopentan-1-carbonsäure, oder ein pharmazeutisch akzeptables Salz derselben.

17. Verbindung nach Anspruch 7, welche darstellt: (*1S*,*2S*,*3R*,*4R*,*1'S*)-(-)-3-(1'-Acetylamino-2'-ethyl)butyl-4-[(N-methylamino-imino)methyl]amino-2-hydroxycyclopentan-1-carbonsäure, oder ein pharmazeutisch akzeptables Salz derselben.

18. Verbindung nach Anspruch 7, welche darstellt: (*1S*,*2S*,*3R*,*4R*,*1'S*)-(-)-3-(1'-Acetylamino-2'-propyl)pentyl-4-[(amino-imino)methyl]amino-2-hydroxycyclopentan-1-carbonsäure, oder ein pharmazeutisch akzeptables Salz derselben.

19. Verbindung nach Anspruch 1, welche darstellt: (*1S*,*2S*,*3R*,*4R*,*1'S*)-(-)-3-(1'-Acetylamino-2'-propyl)pentyl-4-amino-2-hydroxy-cyclopentan-1-carbonsäure, oder ein pharmazeutisch akzeptables Salz derselben.

20. Verbindung nach Anspruch 6, welche darstellt: (*1R*,*2R*,*3S*,*4S*,*1'R*)-(+)-3-(1'-Acetylamino-2'-propyl)pentyl-4-amino-2-hydroxy-cyclopentan-1-carbonsäure, oder ein pharmazeutisch akzeptables Salz derselben.

21. Zusammensetzung zur Hemmung der Neuraminidase des Influenza-Virus', welche einen pharmazeutisch akzeptablen Träger und eine wirksame Menge einer Verbindung nach Anspruch 1 zur Hemmung der Neuraminidase des Influenza-Virus' umfasst.

22. Zusammensetzung zur Behandlung einer Infektion mit dem Influenza-Virus, welche einen pharmazeutisch akzeptablen Träger und eine wirksame Menge einer Verbindung nach Anspruch 1 zur Hemmung der Neuraminidase des Influenza-Virus' umfasst.

## Revendications

1. Composé représenté par la formule : 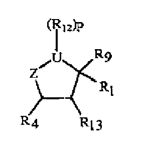 où :
U est CH et p est égal à 1 ;
Z est -C(R₂)(R₃), -CH-N(R₂)(R₃), C(R₃)[(CH₂))ₙR₂], CH-C((R₃)(R₈)(CH₂)ₙR₂, C[(CH₂)ₙR₂]-[CH(R₃)(R₈)], ou C[(R₃)[CH(CH₂)ₙR₂(R₈)];
R1 est H, (CH₂)ₙOH, (CH₂)ₙNH₂, (CH₂)ₙNR₁₀R₁₁, (CH₂)ₙOR₁₁, (CH₂)ₙSR₁₁, ou (CH₂)ₙ halogène ;
R₉ est (CH₂)ₙCO₂H, (CH₂)ₙSO₃H, ou (CH₂)ₙPO₃H₂, leurs esters, ou leurs sels ;
R₂ est NHC(O)R₅, NHC(S)R₅, NHSO₂R₅, C(O)NHR₅, SO₂NHR₅, CH₂S(O)R₅, ou CH₂SO₂R₅ ;
R₃ et R₈ sont individuellement H, (CH₂)ₙC(O)R₁₀, (CH₂)ₙCO₂R₁₀, (CH₂)ₙOR₁₀, CH(OR₁₀)CH(R₁₀)ₘ, C(O)N(R₁₀)ₘ, C(O)N(OR₁₀)R₁₀, (CH₂)ₙN(R₁₀)ₘ, CH(R₁₀)ₘ lorsque m est 2, (CH₂)ₙ(R₁₀)ₘ, CH₂CH(OR₁₀)CH₂OR₁₀, CH(OR₁₀)CH(OR₁₀)CH₂OR₁₀, CH₂OR₁₀, CH(OR₁₀)CH₂NHR₁₀, CH₂CH(OR₁₀)CH₂NHR₁₀, CH(OR₁₀)CH(OR₁₀)CH₂NHR₁₀, C(=NR₁₀)N(R₁₀)ₘ, NHR₁₀, ou NHC(=NR₁₀)N(R₁₀)ₘ ;
R₄ est (CH₂)ₙOH, (CH₂)ₙOR₁₁, (CH₂)ₙOC(O)R₁₁, (CH₂)ₙNHC(=NR₁₁)NHR₁₁, (CH₂)ₙNR₁₀R₁₁, (CH₂)ₙNH₂, (CH₂)ₙC(=NH)(NH2), (CH₂)ₙNHC(=NR₁₁)NH₂, (CH₂)ₙNHC(=NR₇)NH₂, (CH₂)ₙCN, (CH₂)ₙN₃, C (=NH) NH₂, C(=NR₇)NH₂, ou C(=NR₁₁)NH₂ ;
R₅ est H, un alkyle inférieur comportant 1 à 8 atomes de carbone, un cyclo-alkyle, un alkyle substitué par des halogènes, un aryle, un aryle substitué ou CF₃ ;
R₇ est H, (CH₂)ₙOH, (CH₂)ₙCN, (CH₂)ₙNH₂, ou (CH₂)ₙNO₂ ;
R₁₀ est H, un alkyle inférieur comportant 1 à 8 atomes de carbone, un alkenyle inférieur comportant 2 à 8 atomes de carbone, un alkyle ramifié, un alkyle cyclique, un (CH₂)ₙ aromatique, un (CH₂)ₙ aromatique substitué, ou lorsque m est égal à 2 les deux groupes R₁₀ peuvent aussi être connectés pour former un anneau hétérocyclique N substitué, ou un autre anneau hétérocyclique à 5 ou 6 éléments ;
R₁₁ est un alkyle inférieur comportant 1 à 8 atomes de carbone, un alkyle ramifié, un (CH₂)ₘ aromatique, SO₂R₁₀, C(O)R₁₀ ou C(O)OR₁₀ ;
R₁₂ est (CH₂)ₙOH, et R₁₃ est H ;
m est égal à 1 ou 2 ;
n est égal à 0-4 ;
et ses sels acceptables du point du vue pharmaceutique.

2. Composé selon la revendication 1, dans lequel le groupe alkyle inférieur contient 1 à 3 atomes de carbone, et le groupe alkenyle inférieur contient 2 à 3 atomes de carbone.

3. Composé selon la revendication 1, dans lequel le groupe alkyle R₅, R₁₀ et R₁₁ est choisi parmi les groupes méthyle, éthyle, propyle, isopropyle, t-butyle, cyclopentyle, ou cyclohexyle, le groupe aromatique est choisi parmi les groupes aromatiques phényles et alkyles substitués ; le groupe cyclo-alkyle substitué contient 3 à 8 atomes de carbone dans l' anneau et ils sont substitués par 1 ou 2 groupes alkyles comportant 1 à 6 atomes de carbone, un groupe hydroxy ou les deux ; et le groupe alkenyle est choisi parmi les groupes vinyle, 1-propényle, allyle, isopropényle, 2-méthyl-2-propényle et cyclopentyle.

4. Composé selon la revendication 1, dans lequel le sel est celui d'un acide choisi dans le groupe comprenant les acides chlorhydrique, bromhydrique, sulfurique, nitrique, perchlorique, fumarique, maléique, phosphorique, glycolique, lactique, salicylique, succinique, toluène-p-sulfonique, tartrique, acétique, citrique, méthane-sulfonique, formique, benzoïque, malonique, nahpthalène-2-sulfonique, trifluoro-acétique et benzène-sulfonique.

5. Composé selon la revendication 1, dans lequel le sel est un sel de sodium ou d'ammonium lorsque R₉ est (CH₂)ₙCO₂H, (CH₂)ₙSO₃H et (CH₂)ₙPO₃H₂.

6. Composé choisi dans le groupe, comprenant (1S,2S,3R,4R,1'S)-(-)Méthyl 3-(1'acétylamino-2'-éthyl)butyl-4-tert-butoxycarbonyl-amino-2-hydroxycyclopentane-1-carboxylate, (1S,2S,3R,4R,1'S)-(-)-Méthyl 3-(1'-acétylamino-2'-propyl)pentyl-4-tert-butoxycarbonyl-amino-2-hydroxycyclopentane-1-carboxylate, (1S,2S,3R,4R,1'S)-(-)-Ethyl 3- (1'-acétylamino-2'éthyl)butyl-4-tert-butoxycarbonyl-amino-2-hydroxycyclopentane-1-carboxylate, (1S,2S,3R,4R,1'S)-(-)-Méthyl 3-(1'acétylamino-2'-propyl)pentyl-4-tert-butoxycarbonyl-amino-2-hydroxycyclopentane-1-carboxylate, (1R,2R,3S,4S,1'R)-(+)-Ethyl 3-(1'acétylamino-2'-éthyl)butyl-4-tert-butoxycarbonyl-amino-2-hydroxycyclopentane-1-carboxylate, (±)-Méthyl t-3-(1'acétylamino-2'-éthyl)butyl-c-4-tert-butoxycarbonylamino-t-2-hydroxycyclopentane-r-1-carboxylate, (±)-Méthyl t-3-(1'acétylamino-2'-propyl)pentyl-c-4-tert-butoxycarbonylamino-t-2-hydroxycyclopentane-r-1-carboxylate, (±)-Méthyl t-3-(1'-acétylamino-3'-éthyl)pentyl-c-4-tert-butoxycarbonylamino-t-2-acétyloxycyclopentane-r-1-carboxylate, (±)-Méthyl t-3-(1'-acétylamino-n-butyl)-c-4-tert-butoxycarbolylamino-t-2-acétyloxy-cyclopentane-r-1-carboxylate, (±)-Ethyl t-3-(1'acétylamino-2'-éthyl)butyl-c-4-tert-butoxycarbonylamino-t-2-hydroxycyclopentane-r-1-carboxylate, (±)-Ethyl t-3-(1'-acétylamino-2'-méthyl)butyl-c-4-tert-butoxycarbonylamino-t-2-hydroxycyclopentane-r-1-carboxylate, (±)-Ethyl t-3-(1'acétylamino-2'-méthyl)propyl-c-4-tert-butoxycarbonylamino-t-2-hydroxycyclopentane-r-1-carboxylate, hydrochlorure (1S,2S,3R,4R,1'S)-(-)-Méthyl 3-(1'acétylamino-2'-éthyl)butyl-4-amino-2-hydroxy-cyclopentane-1-carboxylate, hydrochlorure (1S,2S,3R,4R,1'S)-(-)-Méthyl 3-(1'-acétylamino-2'-propyl)pentyl-4-amino-2-hydroxycyclopentane-1-carboxylate, trifluoroacétate (1S,2S,3R,4R,1'S)-(-)-Ethyl 3-(1'-acétylamino-2'-éthyl)butyl-4-amino-2-hydroxycyclo-pentane-1-carboxylate, trifluoroacétate (1R,2R,3S,4S,1'R)-(+)-Ethyl 3-(1'-acétylamino-2'-éthyl)butyl-4-amino-2-hydroxy-cyclopentane-1-carboxylate, trifluoroacétate, (±)-Méthyl t-3-(1'-acétylamino-2'-éthyl)butyl-c-4-amino-t-2-hydroxycyclopentane-r-1-carboxylate, trifluoroacétate (±)-Méthyl t-3-(1'-acétylamino-2'-propyl)pentyl-c-4-amino-t-2-hydroxycyclopentane-r-1-carboxylate, trifluoroacétate (±)-Méthyl t-3-(1'-acétylamino-3'-éthyl)pentyl-c-4-amino-t-2-acétyloxycyclopentane-r-1-carboxylate, trifluoroacétate (±)-Méthyl t-3-(1'-acétylamino-n-butyl)-c-4-amino-t-2-acétyloxycyclopentane-r-1-carboxylate, trifluoroacétate (±)-Ethyl t-3-(1'-acétylamino-2'-éthyl)butyl-c-4-amino-t-2-hydroxycyclopentane-r-1-carboxylate, trifluoroacétate (±)-Ethyl t-3-(1'-acétylamino-2'-méthyl) butyl-c-4-amino-t-2-hydroxycyclopentane-r-1-carboxylate, trifluoroacétate (±)-Ethyl t-3-(1'-acétylamino-2'-méthyl)propyl-c-4-amino-t-2-hydroxycyclopentane-r-1-carboxylate, acide (1S,2S,3R,4R,1'S)-(-)-3-(1'-Acétylamino-2'-éthyl)butyl-4-amino-2-hydroxycyclopentane-1-carboxylique, acide (1R,2R,3S,4S,1'R)-(+)-3-(1'-Acétylamino-2'-éthyl)butyl-4-amino-2-hydroxycyclopentane-1-carboxylique, acide (±)-t-3-(1'-Acétylamino-2'-éthyl)butyl-c-4-amino-t-2-hydroxycyclopentane-r-1-carboxylique, acide (±)-t-3-(1'-Acétylamino-2'-propyl)pentyl-c-4-amino-t-2-hydroxycyclopentane-r-1-carboxylique, acide (±)-t-3-(1'-acétylamino-3'-éthyl)pentyl-c-4-amino-t-2-hydroxycyclopentane-r-1-carboxylique, acide (±)-t-3-(1'-acétylamino-n-butyl)-c-4-amino-t-2-hydroxycyclopentane-r-1-carboxylique, acide (±)-t-3-(1'-Acétylamino-2'-méthyl)butyl-c-4-amino-t-2-hydroxycyclopentane-r-1-carboxylique, acide (±)-t-3-(1'-Acétylamino-2'-méthyl)propyl-c-4-amino-t-2-hydroxycyclopentane-r-1-carboxylique, acide(1S,2S,3R,4R,1'S)-(-)-Ethyl 3-(1'-acétylamino-2'-éthyl)butyl-4-{(tert-butoxycarbonyl-amino-tert-butoxycarbonylimino)méthyl}amino-2-hydroxycyclopentane-1-carboxylate, (±)-Méthyl t-3-(1'-acétylamino-2'-éthyl)butyl-c-4-{(tert-butoxycarbonylamino-tert-butoxycarbonylimino)méthyl}amino-t-2-hydroxycyclopentna-r-1-carboxylate, (±)-Méthyl t-3-(1'-acétylamino-3'-éthyl)pentyl-c-4-{(tert-butoxycarbonylamino-tert-butoxycarbonylimino)méthyl}amino-t-2-acétyloxycyclopentane-r-1-carboxylate, (±)-Méthyl t-3-(1'-acétylamino-n-butyl)-c-4-{(tert-butoxycarbonylamino)-tert-butoxycarbonylimino)méthyl}amino-t-2-acétyloxycyclopentane-r-1-carboxylate, (1S,2S,3R,4R,1'S)-(-)Ethyl 3-(1'-acétylamino-2'-éthyl)butyl-4-{(N-tert-butoxycarbonyl-N-méthylamino-N'-tert-butoxycarbonylimino)méthyl}amino-2-hydroxycyclopentane-1-carboxylate, (1S,2S,3R,4R,1'S)-(-)-Ethyl 3-(1'-acétylamino-2'-éthyl)butyl-4-{(amino-imino)méthyl}-amino-2-hydroxycyclopentane-1-carboxylate, (±)-Méthyl t-3-(1'-acétylamino-2'-éthyl)butyl-c-4-{(amino-imino)méthyl}amino-t-2-hydroxycyclopentane-r-1-carboxylate, (±)-Méthyl t-3-(1'-acétylamino-3'-éthyl)pentyl-c-4-{(amino-imino)méthyl}amino-t-2-acétylocyclopentane-r-1-carboxylate, (±)-Méthyl t-3-(1'-acétylamino-n-butyl)-c-4-{(amino-imino)méthyl}amino-t-2-acétyloxycyclopentane-r-1-carboxylate, (1S,2S,3R,4R,1'S)-(-)-Ethyl-3-(1'-acétylamino-2'-éthyl)butyl-4-{(N-méthylamino-imino)méthyl}amino-2-hydroxycyclopentane-1-carboxylate, acide (1S,2S,3R,4R,1'S)-(-)-3-(1'-Acétylamino-2'-éthyl)butyl-4-{(aminoimino)méthyl}amino-2-hydroxycyclopentane-1-carboxylique, acide (±)-t-3-(1'-Acétylamino-2'-éthyl)butyl-c-4-{(aminoimino)méthyl}amino-t-2-hydroxycyclopentane-r-1-carboxylique, acide (±)-t-3-(1'-Acétylamino-3'-éthyl)pentyl-c-4-{(aminoimino)méthyl}amino-t-2-hydroxycyclopentane-r-1-carboxylique, acide (±)-t-3-(1'-Acétylamino-n-butyl)-c-4-{(aminoimino)méthyl}amino-t-2-hydroxycyclopentane-r-1-carboxylique, acide (1S,2S,3R,4R,1'S)-(-)-3-(1'-Acétylamino-2'-éthyl)butyl-4-{(N-méthylamino-imino)méthyl}amino-2-hydroxycyclopentane-1-carboxylique, acide (1S,2S,3R,4R,1'S)-(-)-3-(1'-Acétylamino-2'-propyl)pentyl-4-{(amino-imino)méthyl}amino-2-hydroxycyclopentane-1-carboxylique, et leurs sels acceptables d'un point de vue pharmaceutique.

7. Composé selon la revendication 6, choisi dans le groupe comprenant l'acide (1S,2S,3R,4R,1'S)-(-)-3-(1'-Acétylamino-2'-éthyl)butyl-4-amino-2-hydroxycyclopentane-1-carboxylique, l'acide (±)-t-3-(1'-Acétylamino-2'-éthyl)butyl-c-4-amino-t-2-hydroxycyclopentane-r-1-carboxylique, l'acide (±)-t-3-(1'-Acétylamino-2'-propyl)pentyl-c-4-amino-t-2-hydroxycyclopentane-r-1-carboxylique, l'acide (±)-t-3-(1'-Acétylamino-n-butyl)-c-4-amino-t-2-hydroxycyclopentane-r-1-carboxylique, l'acide (±)-t-3-(1'-acétylamino-2'-méthyl)butyl-c-4-amino-t-2-hydroxycyclopentane-r-1-carboxylique, l'acide (1S,2S,3R,4R,1'S)-(-)-3-(1'-Acétylamino-2'-éthyl)butyl-4-{(aminoimino)méthyl}amino-2-hydroxycyclopentane-1-carboxylique, l'acide (±)-t-3-(1'-Acétylamino-2'-éthyl)butyl-c-4-{(aminoimino)méthyl}amino-t-2-hydroxycyclopentane-r-1-carboxylique, l'acide (±)-t-3-(1'-Acétylamino-3'-éthyl)pentyl-c-4-{(aminoimino)méthyl}amino-t-2-hydroxycyclopentane-r-1-carboxylique, l'acide (±)-t-3-(1'-Acétylamino-n-butyl)-c-4-[(aminoimino)méthyl]amino-t-2-hydroxycyclopentane-r-1-carboxylique, l'acide (1S,2S,3R,4R,1'S)-(-)-3-(1'-Acétylamino-2'-éthyl)butyl-4-{(N-méthylamino-imino)méthyl}amino-2-hydroxycyclopentane-1-carboxylique, acide (1S,2S,3R,4R,1'S)-(-)-3-(1'-Acétylamino-2'-propyl)pentyl-4-amino-imino)méthyl}amino-2-hydroxycyclopentane-1-carboxylique, et leurs sels acceptables d'un point de vue pharmaceutique.

8. Composé selon la revendication 6, constitué de (1S,2S,3R,4R,1'S)-(-)-Méthyl-3-(1'-acétylamino-2'-éthyl)butyl-4-amino-2-hydroxycyclopentane-1-carboxylate, ou d'un sel de celui-ci acceptable d'un point de vue pharmaceutique.

9. Composé selon la revendication 6, constitué de (1S,2S,3R,4R,1'S)-(-)-Méthyl-3-(1'-acétylamino-2'-propyl)pentyl-4-amino-2-hydroxy-cyclopentane-1-carboxylate, ou d'un sel de celui-ci acceptable d'un point de vue pharmaceutique.

10. Composé selon la revendication 6, constitué de (1S,2S,3R,4R,1'S)-(-)-Ethyl-3-(1'-acétylamino-2'-éthyl)butyl-4-amino-2-hydroxy-cyclopentane-1-carboxylate, ou d'un sel de celui-ci acceptable d'un point de vue pharmaceutique.

11. Composé selon la revendication 6, constitué de (1R,2R,3S,4S,1'R)-(+)-Ethyl-3-(1'-acétylamino-2'-éthyl)butyl-4-amino-2-hydroxy-cyclopentane-1-carboxylate, ou d'un sel de celui-ci acceptable d'un point de vue pharmaceutique.

12. Composé selon la revendication 6, constitué d'acide (1S,2S,3R,4R,1'S)-(-)-3-(1'-Acétylamino-2'-éthyl)butyl-4-amino-2-hydroxy-cyclopentane-1-carboxylique, ou d'un sel de celui-ci acceptable d'un point de vue pharmaceutique.

13. Composé selon la revendication 6, constitué d'acide (1R,2R,3S,4S,1'R)-(+)-3-(1'-Acétylamino-2'-éthyl)butyl-4-amino-2-hydroxy-cyclopentane-1-carboxylique, ou d'un sel de celui-ci acceptable d'un point de vue pharmaceutique.

14. Composé selon la revendication 6, constitué de (1S,2S,3R,4R,1'S)-(-)Ethyl-3-(1'-acétylamino-2'-éthyl)butyl-4-[(aminoimino)méthyl]amino-2-hydroxycyclopentane-1-carboxylate, ou d'un sel de celui-ci acceptable d'un point de vue pharmaceutique.

15. Composé selon la revendication 6, constitué de (1S,2S,3R,4R,1'S)-(-)-Ethyl-3-(1'-acétylamino-2'-éthyl)butyl-4-[(N-méthylamino-imino)méthyl]amino-2-hydroxycyclopentane-1-carboxylate, ou d'un sel de celui-ci acceptable d'un point de vue pharmaceutique.

16. Composé selon la revendication 7, constitué d'acide (1S,2S,3R,4R,1'S)-(-)-3-(1'-Acétylamino-2'-éthyl)butyl-4-[(aminoimino)méthyl]-amino-2-hydroxycyclopentane-1-carboxylique, ou d'un sel de celui-ci acceptable d'un point de vue pharmaceutique.

17. Composé selon la revendication 7, constitué d'acide (1S,2S,3R,4R,1'S)-(-)-3-(1'-Acétylamino-2'-éthyl)butyl-4-[(N-méthylamino-imino)méthyl]-amino-2-hydroxycyclopentane-1-carboxylique, ou d'un sel de celui-ci acceptable d'un point de vue pharmaceutique.

18. Composé selon la revendication 7, constitué d'acide (1S,2S,3R,4R,1'S)-(-)-3-(1'-Acétylamino-2'-propyl)pentyl-4-[(aminoimino)-méthyl]-amino-2-hydroxycyclopentane-1-carboxylique, ou d'un sel de celui-ci acceptable d'un point de vue pharmaceutique.

19. Composé selon la revendication 1, constitué d'acide (1S,2S,3R,4R,1'S)-(-)-3-(1'-Acétylamino-2'-propyl)pentyl-4-amino-2-hydroxy-cyclopentane-1-carboxylique, ou d'un sel de celui-ci acceptable d'un point de vue pharmaceutique.

20. Composé selon la revendication 6, constitué d'acide (1R,2R,3S,4S,1'R)-(+)-3-(1'-Acétylamino-2'-propyl)pentyl-4-amino-2-hydroxy-cyclopentane-1-carboxylique, ou d'un sel de celui-ci acceptable d'un point de vue pharmaceutique.

21. Composition pour inhiber la neuraminidase du virus influenza, comprenant un porteur acceptable d'un point de vue pharmaceutique et une quantité d'un composé selon la revendication 1 efficace pour inhiber la neuraminidase du virus influenza.

22. Composition pour traiter une infection par le virus influenza, comprenant un porteur acceptable d'un point de vue pharmaceutique et une quantité d'un composé selon la revendication 1 efficace pour inhiber la neuraminidase du virus influenza.
